(19) **Europäisches Patentamt / European Patent Office / Office européen des brevets**

(11) **EP 1 651 313 B1**

(12) **EUROPÄISCHE PATENTSCHRIFT**

(45) Veröffentlichungstag und Bekanntmachung des Hinweises auf die Patenterteilung:
**08.04.2009 Patentblatt 2009/15**

(21) Anmeldenummer: **04741010.5**

(22) Anmeldetag: **14.07.2004**

(51) Int Cl.:
*A61Q 5/06* (2006.01)   *A61K 8/81* (2006.01)

(86) Internationale Anmeldenummer:
**PCT/EP2004/007801**

(87) Internationale Veröffentlichungsnummer:
**WO 2005/012389 (10.02.2005 Gazette 2005/06)**

(54) **VERFAHREN ZUR HAARBEHANDLUNG MIT FORMGEDÄCHTNISPOLYMEREN**

METHOD FOR TREATING HAIR WITH SHAPE MEMORY POLYMERS

PROCEDE DE TRAITEMENT DE CHEVEUX AU MOYEN DE POLYMERES A MEMOIRE DE FORME

(84) Benannte Vertragsstaaten:
**AT BE BG CH CY CZ DE DK EE ES FI FR GB GR HU IE IT LI LU MC NL PL PT RO SE SI SK TR**

(30) Priorität: **30.07.2003 DE 10334823**

(43) Veröffentlichungstag der Anmeldung:
**03.05.2006 Patentblatt 2006/18**

(73) Patentinhaber: **Mnemoscience GmbH**
**52531 Uebach-Palenberg (DE)**

(72) Erfinder:
 • **KRAUSE, Thomas**
  **64297 Darmstadt (DE)**
 • **KALBFLEISCH, Axel**
  **64295 Darmstadt (DE)**
 • **DUCHSCHERER, Anja**
  **65589 Hadamar (DE)**
 • **BEYER, Angelika**
  **63857 Waldaschaff (DE)**
 • **GRANER, Susanne**
  **64331 Weiterstadt (DE)**
 • **HASSON, Ali Abdulla Tareq**
  **52062 Aachen (DE)**
 • **LENDLEIN, Andreas**
  **14167 Berlin (DE)**
 • **KRATZ, Karl**
  **10115 Berlin (DE)**
 • **JIANG, Hong-Yan**
  **52062 Aachen (DE)**

 • **MÖRSHEIM, Simone**
  **52249 Eschweiler (DE)**
 • **Weber, Yasmin**
  **28213 Bremen (DE)**

(74) Vertreter: **Grünecker, Kinkeldey, Stockmair & Schwanhäusser Anwaltssozietät**
**Leopoldstrasse 4**
**80802 München (DE)**

(56) Entgegenhaltungen:
**WO-A-99/42147**   **US-A- 5 618 850**
**US-A1- 2003 124 074**   **US-B1- 6 203 802**

 • **YOUNG KWEON CHOI, YOU HAN BAE, SUNG WAN KIM: "Star-Shaped Poly(ether-ester) Block Copolymers: Synthesis, Characterization and Their Physical Properties" MACROMOLECULES, Bd. 31, 26. November 1998 (1998-11-26), Seiten 8766-8774, XP002305027**
 • **PATENT ABSTRACTS OF JAPAN Bd. 016, Nr. 213 (C-0942), 20. Mai 1992 (1992-05-20) -& JP 04 041416 A (NOEVIR CO LTD; others: 01), 12. Februar 1992 (1992-02-12) in der Anmeldung erwähnt**
 • **LENDLEIN A ET AL: "Formgedächtnispolymere" ANGEWANDTE CHEMIE, VCH VERLAGSGESELLSCHAFT, WEINHEIM, DE, Bd. 114, Nr. 12, 17. Juni 2002 (2002-06-17), Seiten 2138-2162, XP002251533 ISSN: 0044-8249**

**Beschreibung**

Gegenstand der vorliegenden Erfindung ist ein Verfahren zur Erzielung einer abrufbaren Haarumformung unter Verwendung von Formgedächtnispolymeren.

Bei der Formgebung von Haaren wird im allgemeinen zwischen temporärer und dauerhafter, permanenter Haarverformung unterschieden. Eine temporäre Haarverformung erfolgt in der Regel unter Verwendung von Zusammensetzungen auf Basis von Lösungen oder Dispersionen haarfestigender Polymere. Derartige Produkte verleihen den Haaren durch den Polymerzusatz mehr oder weniger Halt, Volumen, Elastizität, Sprungkraft und Glanz. Diese Stylingprodukte erleichtern z.B. als Gel die Formgebung und Erstellung der Frisur, verbessern als Haarspray den Stand einer erstellten Frisur und erhöhen als Festigerschaum das Volumen des Haares. Nachteilig ist, dass die gewünschten Effekte nur von relativ kurzer Dauer sind und durch äußere Einflüsse wie Kämmen, Wind, hohe Luftfeuchtigkeit oder Kontakt mit Wasser rasch wieder verloren gehen. Eine permanente Haarverformung erfolgt in der Regel durch eine Dauerwellbehandlung. Hierbei werden Disulfidbindungen im Haar reduktiv gespalten, das Haar in die neue Form gebracht und durch oxidative Bildung neuer Disulfidbindungen fixiert. Nachteilig ist, dass durch die erforderliche chemische Behandlung des Haares mit Reduktions- und Oxidationsmitteln eine Beeinträchtigung der Haarstruktur nicht vermieden werden kann. Ein weiterer Nachteil der bisher bekannten Verfahren zur Haarumformung ist, dass es nicht möglich ist, eine Umformung in relativ einfacher Weise rückgängig zu machen, d.h. von einer Frisurenform ohne aufwändige Neuerstellung zu einer anderen zu gelangen.

Aus der JP 04-41416 sind Haarkosmetika bekannt, welche bestimmte lineare Polyurethane mit einer Glasübergangstemperatur $T_g$ von 40-90°C enthalten. Das beschriebene Verfahren zur Haarbehandlung entspricht einer Behandlung mit einem typischen Thermoplasten. Nach Auftragen der Zusammensetzung wird oberhalb von $T_g$ die Frisurenform erstellt und durch Abkühlen unter $T_g$ fixiert. Bei erneutem erwärmen oberhalb $T_g$ erweicht das Polymer und eine neue Frisur kann erstellt werden. Ein Verfahren für eine abrufbare, reversible Haarumformung wird nicht beschrieben. Die Eigenschaften der linearen Polyurethane sind für eine Anwendung zur abrufbaren Haarumformung unzureichend.

Die der vorliegenden Erfindung zugrunde liegende Aufgabe bestand darin, ein Verfahren und die hierfür erforderlichen Produkte für eine abrufbare Haarverformung mit hohem Wiederherstellungsgrad einer programmierten Frisurenform zur Verfügung zu stellen. Eine weitere Aufgabe bestand darin, ein Verfahren zur Verfügung zu stellen, mit dem es möglich ist, eine dauerhafte Haarumformung ohne schädigenden Eingriff in die Haarstruktur zu erreichen. Eine weitere Aufgabe bestand darin, ein Verfahren zur Verfügung zu stellen, welches ermöglicht, temporäre Umformungen mehrfach in einfacher Weise rückgängig zu machen und mit hoher Genauigkeit zu einer zuvor erstellten, programmierten, permanenten Frisurenform zurückzukehren. Eine weitere Aufgabe bestand darin, ein Verfahren zur Verfügung zu stellen, welches ermöglicht, in einfacher Weise und mit hoher Genauigkeit auf äußere Einflüsse zurückzuführende Deformationen einer Frisur rückgängig zu machen und zu einer zuvor erstellten, programmierten, permanenten Frisurenform zurückzukehren.

Die Aufgabe wird gelöst durch ein Verfahren zur Haarbehandlung, wobei

- eine Zusammensetzung, welche mindestens ein Formgedächtnispolymer P und/oder mindestens ein vernetzbares Makromer M, welches nach Vernetzung ein Formgedächtnispolymer bildet, enthält, auf das Haar aufgebracht wird, wobei das Polymer P und das Makromer M gebildet werden aus Blockpolymeren aus jeweils mindestens einem ersten Block, bei dem es sich um ein Polyol handelt, ausgewählt aus Polyethern, Oligoethern, Kohlenwasserstoffen mit einem Molekulargewicht von mindestens 400 g/mol und mindestens zwei alkoholischen Hydroxygruppen, Oligoesterdiolen und Polyestern aus Dicarbonsäuren mit Diolen; und mindestens zwei weiteren Blöcken, bei denen es sich um Polyester von Hydroxycarbonsäuren oder deren Lactonen handelt, wobei das Makromer M

   a) vernetzbare Bereiche enthält, die durch chemische Bindungen vernetzbar sind und
   b) thermoplastische Bereiche enthält, die nicht chemisch vernetzbar sind und
   c) das nach Vernetzung gebildete Formgedächtnispolymer mindestens eine Übergangstemperatur $T_{trans}$ aufweist;

   und wobei das Polymer P

   a) mindestens ein durch physikalische Wechselwirkung vernetzbares hartes Segment mit einer ersten Übergangstemperatur $T'_{trans}$, die oberhalb Raumtemperatur liegt, und
   b) mindestens ein weiches Segment mit einer zweiten Übergangstemperatur $T_{trans}$, welche unterhalb von $T'_{trans}$ liegt, aufweist;

- vorher, gleichzeitig oder anschließend das Haar in eine bestimmte (permanente) Form gebracht wird und
- anschließend die Form durch chemische Vernetzung des Makromers unter Ausbildung eines Formgedächtnispolymers und/oder unter physikalischer Vernetzung des Polymers P fixiert wird; wobei die programmierte Frisur (permanente Form) auf eine Temperatur oberhalb $T_{trans}$ erwärmt wird, das Haar in eine zweite (temporäre) Form gebracht wird und die zweite Form durch Abkühlen auf eine Temperatur unterhalb $T_{trans}$ fixiert wird.

Ein Polyol im Sinne der Erfindung ist eine Verbindung, welche mindestens zwei alkoholische Hydroxygruppen aufweist. Die Kohlenwasserstoffe können linear, verzweigt, gesättigt, ein- oder mehrfach ungesättigt sein. Poly- und Oligoether sind polymere bzw. oligomere Verbindungen, deren organische Wiederholungseinheiten durch Ether-Funktionalitäten (C-O-C) zusammengehalten werden. Im Sinne der Erfindung haben Polyether mindestens 4 Ethergruppen und mindestens 5 Wiederholungseinheiten und Oligoether eine, zwei oder drei Ethergruppen und 2 bis 4 Wiederholungseinheiten.

Ein Gegenstand der Erfindung ist ein Verfahren zur Aufprägung einer zweiten Frisurenform auf eine einprogrammierte, abrufbare erste Frisurenform. Hierbei wird zunächst eine durch das oben genannte Verfahren programmierte Frisur (permanente Form) auf eine Temperatur oberhalb $T_{trans}$ erwärmt. Anschließend wird das Haar in die gewünschte zweite (temporäre) Form gebracht und die zweite Form wird durch Abkühlen auf eine Temperatur unterhalb $T_{trans}$ fixiert.

Ein weiterer Gegenstand der Erfindung ist ein Verfahren zur Wiederherstellung einer zuvor durch das oben genannte Verfahren einprogrammierten ersten Frisur (permanente Form). Hierfür wird eine Frisur in einer temporären Form auf eine Temperatur oberhalb $T_{trans}$ erwärmt. Die permanente Form bildet sich dabei spontan und selbsttätig zurück.

Frisur oder Frisurenform im Sinne der Erfindung ist breit zu verstehen und umfaßt beispielsweise auch den Grad der Wellung oder den Grad der Glattheit von Haaren. Eine programmierte Frisur im Sinne der Erfindung ist eine Ansammlung von Haaren, die durch vernetzte und in einer permanenten Form fixierte Formgedächtnispolymere eine bestimmte Form aufweisen. Wiederherstellung einer programmierten Frisur im Sinne der Erfindung bedeutet, dass sich die programmierte Frisur nach einer Deformation wieder zu vorzugsweise mindestens 60%, besonders bevorzugt mindestens 80% zurückbildet, bezogen auf die Form, die nach einem ersten Relaxationszyklus entsteht. Der Grad der Wiederherstellung kann beispielsweise durch Längenmessung einer Haarlocke oder einer Haarsträhne erfolgen.

Unter Kaltverformung einer Frisur ist eine Frisurenänderung bei Umgebungstemperatur, ohne Zuführung von zusätzlicher Wärme durch einen Haartrockner oder ähnliche Geräte zu verstehen. Die Verformung kann dabei mechanisch verursacht sein, z.B. durch bloßes Aushängen der Locken unter Schwerkrafteinwirkung, durch Kämmen oder Bürsten der Haare, durch Wind oder Feuchtigkeit, durch mechanische Einflüsse während des Schlafens oder Liegens etc..

Formgedächtnispolymere im Sinne der Erfindung sind Polymere, aus denen sich Materialien herstellen lassen mit der Eigenschaft, dass sich ihnen eine beliebige Form (permanente Form) aufprägen läßt, in die sie sich nach einer Deformation oder nach Aufprägen einer zweiten Form (temporäre Form) spontan und ohne äußere Krafteinwirkung durch blosses Erwärmen oder durch einen anderen energetischen Stimulus zurückverwandeln. Deformation und Rückverwandlung (recovery) sind dabei mehrfach möglich. Der Grad der Erreichung der ursprünglichen, permanenten Form ist bei einem ersten Relaxationszyklus, bestehend aus Deformation und Rückverwandlung, in der Regel etwas geringer als bei nachfolgenden Zyklen, vermutlich wegen der Beseitigung von anfänglich noch vorhanden Fehlstellen, Texturen etc.. Ein besonders hoher Rückverwandlungsgrad wird dann aber bei den nachfolgenden Relaxationszyklen erreicht. Der Grad der Rückverwandlung beträgt beim ersten Relaxationszyklus vorzugsweise mindestens 30%, besonders bevorzugt mindestens 50% und bei den nachfolgenden Relaxationszyklen vorzugsweise mindestens 60%, besonders bevorzugt mindestens 80%. Er kann aber auch 90% und mehr betragen. Der Grad der Rückverwandlung kann gemessen werden wie bei üblichen curl retention Messungen durch einfache Längenmessung einer behandelten Haarlocke oder durch bekannte, geeignete Zug-Dehnungs-Experimente.

Physikalisch vernetzbare Formgedächtnispolymere im Sinne der Erfindung sind Polymere, bei denen das Fixieren der aufgeprägten permanenten Form durch Vernetzung aufgrund von physikalischen Wechselwirkungen erfolgt. Eine Vernetzung durch physikalische Wechselwirkungen kann dadurch erfolgen, dass sich bestimmte Segmente der Polymerketten zu kristallinen Bereichen zusammenlagern. Bei den physikalischen Wechselwirkungen kann es sich um charge transfer Komplexe, um Wasserstoffbrückenbindungen, um dipolare oder hydrophobe Wechselwirkungen, um van der Waals-Wechselwirkungen oder um ionische Wechselwirkungen von Polyelektrolytsegmenten handeln. Die Wechselwirkungen können zwischen verschiedenen Segmenten innerhalb eines Polymerstranges (intramolekular) und/oder zwischen verschiedenen Polymersträngen (intermolekular) erfolgen. Die Ausbildung der Wechselwirkungen kann beispielsweise durch Abkühlen (insbesondere im Falle von Kristallisationen) und/oder durch Trocknen, d.h. durch Entfernen von Lösungsmitteln ausgelöst werden.

Erfindungsgemäß geeignete physikalisch vernetzbare Formgedächtnispolymere bestehen aus mindestens einem harten Segment und mindestens einem weichen Segment und weisen mindestens zwei Übergangstemperaturen $T_{trans}$ und $T'_{trans}$ auf. Die Polymersegmente sind vorzugsweise Oligomere oder Dihydroxykohlenwasserstoffe, insbesondere lineare Kettenmoleküle mit einem Molekulargewicht von beispielsweise 400 bis 30000, vorzugsweise 1000 bis 20000 oder 1500 bis 15000. Das Molekulargewicht der Polymere kann beispielsweise von 30000 bis 1000000, vorzugsweise von 50000 bis 700000 oder von 70000 bis 400000 betragen. Sie weisen einen Kristallinitätsgrad von vorzugsweise 3 bis 80%, besonders bevorzugt von 3 bis 60% auf. Bei beiden Übergangstemperaturen kann es sich z.B. um Schmelztemperaturen $T_m$ oder um Glasübergangstemperaturen $T_g$ handeln. Oberhalb von $T_{trans}$ weist das Polymer ein niedrigeres Elastizitätsmodul auf als unterhalb von $T_{trans}$. Das Verhältnis der Elastizitätsmodule unter- und oberhalb von $T_{trans}$ ist vorzugsweise mindestens 10, besonders bevorzugt mindestens 20. Die untere Übergangstemperatur $T_{trans}$ ist vorzugsweise größer als Raumtemperatur (20°C), insbesondere mindestens 30°C, besonders bevorzugt mindestens 35°C oder mindestens 40°C und ist diejenige Temperatur, bei deren Überschreiten die spontane Rückbildung der permanten Form aus der deformierten oder aus der temporären Form erfolgt. $T_{trans}$ liegt vorzugsweise soweit oberhalb von gewöhnlich zu erwartenden Umgebungstemperaturen, dass bei Umgebungstemperatur keine signifikante, unbeabsichtigte, thermisch induzierte Verformung der temporären Frisurenform auftritt. Geeignete Bereiche für $T_{trans}$ sind z.B. von 25 bis 100°C, von 30 bis 75°C, von von 35 bis 70°C oder von 40 bis 60°C. Die obere Übergangstemperatur $T'_{trans}$ liegt über $T_{trans}$ und ist diejenige Temperatur, oberhalb der die Aufprägung der permanenten Form oder die Umprägung einer permanenten Form in eine neue permanente Form erfolgt und durch deren Unterschreiten die permanente Form fixiert wird. $T'_{trans}$ liegt vorzugsweise soweit oberhalb von $T_{trans}$, dass bei Erwärmung der Frisur auf eine Temperatur oberhalb $T_{trans}$ zur Wiederherstellung der permanenten Frisurenform oder zur Neuerstellung einer temporären Frisurenform unter Beibehaltung der permanenten Frisurenform keine signifikante, unbeabsichtigte, thermisch induzierte Verformung der permanenten Frisurenform auftritt. Vorzugsweise liegt $T'_{trans}$ mindestens 10°C, besonders bevorzugt mindestens 20°C oder mindestens 30°C oberhalb $T_{trans}$. Die Differenz zwischen $T'_{trans}$ und $T_{trans}$ kann beispielsweise von 10 bis 80°C, von 20 bis 70°C oder von 30 bis 60°C betragen. Geeignete Bereiche für $T'_{trans}$ sind z.B. von 40 bis 150°C, von 50 bis 100°C oder von 70 bis 95°C.

Die Formgebung der Haare erfolgt zweckmäßigerweise unter Erwärmung auf eine Temperatur von mindestens $T'_{trans}$ und die Haarform wird durch Abkühlung auf eine Temperatur unterhalb $T'_{trans}$ fixiert. Raumtemperatur bedeutet in der Regel Umgebungstemperatur, vorzugsweise mindestens 20°C, bei wärmerem Klima vorzugsweise mindestens 25°C. Das Aufbringen der Zusammensetzung auf das Haar kann auf verschiedene Weisen erfolgen, z.B. direkt durch Versprühen oder indirekt durch Aufbringen zunächst auf die Hand oder auf ein geeignetes Hilfsmittel wie z.B. Kamm, Bürste etc. und anschließendem Verteilen im bzw. auf dem Haar. Die Konsistenz der Zusammensetzung kann beispielsweise diejenige sein einer Lösung, Dispersion, Lotion, verdickten Lotion, Gel, Schaum, einer halbfesten Masse, cremeartig oder wachsartig.

Geeignete Polymere P sind z.B. solche der allgemeinen Formel

$$A(B)_n \qquad (I)$$

wobei A von einem n-valenten Poly- oder Oligoether, Kohlenwasserstoffen mit einem Molekulargewicht von mindestens 400 g/mol und n alkoholischen Hydroxygruppen, Oligoesterdiolen oder von einem Polyester einer Dicarbonsäure mit einem Diol, oder von einem Dimerdiol abgeleitet ist, B einen Poly(hydroxycarbonsäure)block, und n eine Zahl größer gleich zwei, vorzugsweise 2, 3 oder 4 bedeuten. Bevorzugt sind B-A-B Triblockpolymere, insbesondere Blockpolymere mit einem einzigen Polyolblock als Mittelblock, zwei endständigen Polyesterblöcken aus Hydroxycarbonsäuren oder deren Lactonen sowie mit endständigen alkoholischen Hydroxygruppen.

A steht bevorzugt für Polyalkylenglykolether von mehrwertigen Alkoholen, Poly(tetrahydrofuran), Dimerdiol, Dimerdiololigoethern und Oligoesterdiolen (Dihydroxyoligoester). Die Polyalkylenglykolether haben vorzugsweise 2 bis 6 C-Atome pro Alkylengruppe, besonders bevorzugt sind Poly(ethylenglycol) (PEG) und Poly(propylenglycol) (PPG). Dimeriol ist die Bezeichnung für alpha, omega-C36-Diole, die durch Dimerisierung von Oleylalkohol oder durch Hydrierung von Dimerfettsäuren hergestellt werden können. Dimerfettsäure ist eine Mischung aliphatischer, verzweigter oder cyclischer C36-Dicarbonsäuren (Dimersäure), herstellbar durch Dimerisierung von Oleinsäure oder Tallölfettsäure (TOFA). Dimerdiol ist erhältlich z.B. unter der Handelsbezeichnung Sovermol® 908. Dimerdiololigoether sind Oligomere von Dimerdiol, herstellbar durch säurekatalysierte Dehydratisierung von Dimerdiol. Bevorzugt sind Dimere, Trimere und Tetramere von Dimerdiol. Geeignete Handelsprodukte sind z.B. Sovermol® 909 mit einem Molekulargewicht von ca. 1000 oder Sovermol® 910 mit einem Molekulargewicht von ca. 2000.

Oligoesterdiole (Dihydroxyoligoester) sind Reaktionsprodukte einer Oligomerisierung einer Dicarbonsäure mit einem Diol, wobei das Reaktionsprodukt zwei Hydroxygruppen aufweist. Geeignete Dicarbonsäure sind beispielsweise C3- bis C20-Dicarbonsäuren, vorzugsweise aliphatische C4- bis C10-Dicarbonsäuren. Aliphatische Dicarbonsäuren sind z.B. Oxalsäure, Malonsäure, bernsteinsäure, Glutarsäure, Adipinsäure, Pimelinsäure, Azelainsäure, Sebacinsäure, Maleinsäure, Fumarsäure. Aromatische Dicarbonsäure sind z.B. Phtalsäure und Terephtalsäure. Diolkomponenten der Oligoesterdiole sind z.B. C2- bis C30-Diole, vorzugsweise aliphatische C5- bis C20-Diole. Geeignet sind auch Glycerinmonoester, insbesondere Monoester von Glycerin mit aliphatischen C2- bis C30-Monocarbonsäuren, vorzugsweise C5- bis C20-Monocarbonsäuren.

Der Poly(hydroxycarbonsäure)block B kann gebildet werden aus Hydroxycarbonsäuren, insbesondere Monohydroxymonocarbonsäuren, die bis zu 30 C-Atome aufweisen können sowei aus deren Lactonen oder Lactiden. Der Poly(hydroxycarbonsäure)block B kann auch ein Copolymer aus zwei oder mehr verschiedenen Hydroxycarbonsäuren sein. Hydroxycarbonsäuren können sein: gesättigte oder ungesättigte aliphatische Hydroxycarbonsäuren, aromatische Hydroxycarbonsäuren, alpha-Hydroxycarbonsäuren, beta-Hydroxycarbonsäuren, omega-Hydroxycarbonsäuren, Hydroxyfettsäuren. Alpha-Hydroxycarbonsäuren sind z.B. Glykolsäure, Milchsäure oder Mandelsäure. Beta-Hydroxycarbonsäuren sind z.B. beta-Hydroxyalkansäuren wie z.B. beta-Hydroxybuttersäure oder beta-Hydroxyvaleriansäure. Hydroxyfettsäuren sind z.B. 12-Hydroxystearinsäure oder Ricinolsäure. Aromatische Hydroxysäuren sind beispielsweise Hydroxybenzoesäuren, z.B. Salicylsäure.

B steht bevorzugt für Poly($\varepsilon$-caprolacton), Polylactiden, Polyglycoliden, Poly(lactid-co-glycolid), Poly(pentadecalacton), Poly(caprolacton-co-lactid), Poly(pentadecalacton-co-lactid), Poly(pentadecalacton-co-caprolacton). Ein insbesondere bevorzugter, erfindungsgemäß einzusetzender Polyester ist ein Polyester auf der Basis von Lactideinheiten oder Pentadecalactoneinheiten.

Bevorzugte Blockcopolymere sind solche der allgemeinen Formel

$$\text{HO- } [\text{B1-C (=O)O-}]_{n1} [\text{Y-O-}]_{n2} [\text{C (=O) -B2-O-}]_{n3}\text{H} \qquad\qquad \text{(Ia)}$$

B1 und B2 sind gleich oder verschieden und stehen für verzweigte, cyclische oder lineare Alkylengruppen mit 1 bis 40, vorzusgweise mit 2 bis 20 C-Atomen. Y steht für eine verzweigte, cyclische oder lineare Alkylengruppe mit 2 bis 30 C-Atomen, vorzugsweise für Ethylen- und/oder Propylengruppen, oder für einen Block aus Dimerdiol, Dimerdioloigoether oder Oligoesterdiol, und wobei n1, n2 und n3 gleiche oder verschiedene Zahlen größer oder gleich Null sind, wobei sowohl n2 als auch die Summe n1+n3 größer als Null sind.

Besonders bevorzugt sind Polymere der Formel (Ia), wobei B1 und B2 für verzweigte oder lineare Alkylengruppen mit 2 bis 20 C-Atomen stehen, Y eine Ethylengruppe ist und n1, n2 und n3 größer Null sind und so gewählt sind, dass das Molekulargewicht des Polymers größer oder gleich 2000 ist. Geeignet sind z.B. Blockcopolymere mit einem Polyethylenglykolmittelblock und Endblöcken aus Polymilchsäure oder Poly-$\varepsilon$-caprolacton, wobei die Endblöcke an den Enden mit Hydroxygruppen substituiert sind, der Mittelblock ein Molekulargewicht im Bereich von 500 bis 20000, vorzugsweise 2000 bis 10000 aufweist und das mittlere Molekulargewicht des Blockcopolymers von 2000 bis 50000, vorzugsweise von 3000 bis 25000 beträgt.

Der Vorteil der Copolymere mit Blockstruktur ist, dass sich die unterschiedlichen Eigenschaften und Funktionen der Blöcke in einem Polymer kombinieren lassen. Die Eigenschaften der Blöcke, wie z.B. die Hydrophilie, die Affinität zum Haar oder die Schalttemperatur, lassen sich dabei unabhängig voneinander einstellen.

Zur Herstellung der Blockcopolymere kann beispielsweise ein oligomeres oder polymeres Diol als difunktioneller Initiator für eine ringöffnende Polymersiarion (ROP) verwendet werden. Der Initiator stellt dann den A-Block dar. Als Initiator werden bevorzugt Polyetherdiole eingesetzt, welche in verschiedenen Molgewichten kommerziell erhältlich sind. Bevorzugt verwendet wird PEO oder PEG mit einem Molgewicht von 4000 bis 8000 g/mol, besonders bevorzugt mit einem Molgewicht von 6000 und 8000 g/mol, was der A-Blocklänge entspricht.

Weitere, bevorzugte Diole sind

- Dimerdiol (Dimerfettdiol), kommerziell erhältlich unter dem Handelsnamen Sovermol® 908, mit einem Molekulargewicht von 550 g/mol. Die Herstellung von Dimerdiol ist bekannt. Dimerdiol kann beispielsweise durch Hydrierung von dimerer Ölsäure und/oder deren Ester gemäß der deutschen Patentschrift DE-B 17 68 313 hergestellt werden. Die allgemeine Formel lautet $\text{HO-CH}_2\text{-C}_{34}\text{H}_{66}\text{-CH}_2\text{-OH}$.

- Dimerdioloigoether, kommerziell erhältlich unter den Handelsnamen Sovermol® 909, mit einem Molekulargewicht von ca. 1000 g/mol, und Sovermol® 910, mit einem Molekulargewicht von ca. 2000 g/mol. Die allgemeine Formel lautet $\text{HO-CH}_2\text{-C}_{34}\text{H}_{66}\text{-CH}_2\text{-O-}[\text{CH}_2\text{-C}_{34}\text{H}_{66}\text{-CH}_2\text{-O-}]_n\text{H}$ wobei n eine Zahl von 1 bis 5, vorzugsweise 1, 2 oder 3 ist.

- Oligoesterdiole mit Molekulargewichten zwischen 1000 und 6000 g/mol, die ausgehend von Dicarbonsäuren und Diolen auf Fettbasis synthetisiert wurden. Bei den Diolen auf Fettbasis handelt es sich insbesondere um lineare oder verzweigte aliphatische $C_2$- bis $C_{14}$-Diole, 12-Hydroxystearylalkohol, Dimerfettdiol, Dimerfettdioloigoether mit einem Molekulargewicht von 1000 bis 2000, und Glycerinmonoester mit Oleinsäure, Stearinsäure oder Laurinsäure. Bei den Dicarbonsäuren handelt es sich vorzugsweise um Dimerfettsäure, Adipinsäure und Azelainsäure.

Die Oligoesterdiole der folgenden allgemeinen Formeln sind geeignet:

Oligoesterdiole des Dimerdiols:

$$\text{HO-CH}_2\text{-C}_{34}\text{H}_{66}\text{-CH}_2\text{-O-}[(\text{O=})\text{C-R-C(=O)-O-CH2-C}_{34}\text{H}_{66}\text{-CH}_2\text{-O-}]_n\text{H}$$

Oligoesterdiole des 12-Hydroxystearylalkohols:

$$HO\text{-}(CH_2)_{11}\text{-}CH(C_6H_{13})\text{-}O\text{-}f(O=)C\text{-}R\text{-}C(=O)\text{-}O\text{-}(CH_2)_{11}\text{-}CH(C_6H_{13})\text{-}O\text{-}I_nH$$

Insbesondere bevorzugt sind Hydroxystearylalkohol-adipat-diol und Hydroxystearylalkohol-azeleat-diol. Oligoesterdiole des Glycerolmonostearats:

$$HO\text{-}CH(CH_2\text{-}O\text{-}C(=O)(C_{17}H_{35}))\text{-}CH_2O\text{-}[(O=)C\text{-}R\text{-}C(=O)\text{-}O\text{-}CH_2\text{-}CH\text{-}(CH_2O\text{-}C$$
$$(=O)\text{-}(C_{17}H_{35}))\text{-}CH_2O\text{-}]_nH$$

In allen Formeln steht R für lineare, aliphatische Kohlenwasserstoffe mit 4 bis 36 C-Atomen und n für Zahlen größer ode gleich 1, vorzugsweise größer oder gleich 2.

Die Herstellung von Oligoesterdiolen aus Diolen und Dicarbonsäuren ist im Stand der Technik bekannt. Die Herstellung kann mit oder ohne Einsatz von Katalysatoren erfolgen. Als Katalysatoren können alle Veresterungskatalysatoren wie z. B. Schwefelsäure, Phosphorsäure oder p-Toululsulfonsäure verwendet werden. Auch Zinn-Verbindungen wie Zinndioctoat, Zinnoxid und Zinnoxalat bzw. Titanverbindungen wie z.B. Titan(IV)isopropoxid können in Frage kommen .
Bevorzugt sind p-Toluolsulfonsäure und
Titan(IV)isopropoxid.
Zur Einführung der B-Blöcke können zyklische Ester oder Diester verwendet werden, wie beispielsweise Dilactid, Diglycolid, p-Dioxanon, ε-Caprolacton, ω-Pentadecalacton oder deren Mischungen. Bevorzugt verwendet wird Dilactid, *L,L*-Dilactid oder ω-Pentadecalacton. Die Reaktion erfolgt bevorzugt in der Masse, optional unter Zusatz eines Katalysators, wie beispielsweise Dibutylzinn(IV)oxid, Dibutylzinn(IV)dilaurat, Zinn(II)dilaurat, Zinn(II)octanoat, Titan(IV) isopropoxid oder Lithiumchlorid. Bevorzugt sind Dibutylzinn(IV)oxid und Zinn(II)octanoat, insbesondere Dibutylzinn(IV) oxid. Wenn ein Katalysator eingesetzt wird, dann kann das in Mengen von 0,1 bis 0,3 mol% erfolgen. Da viele der möglichen Katalysatoren, insbesondere die Zinnverbindungen toxisch sind, muss bei einem Einsatz der Triblockcopolymere in Materialien für den kosmetischen oder medizinischen Bereich der im Copolymer verbleibende Katalysatorrest entfernt werden. Die jeweiligen Verfahrensbedingungen sind dem Fachmann bekannt und durch die nachfolgenden Beispiele illustriert.
Die B-Blocklänge kann über das molare Verhältnis von Monomer zu Initiator variabel eingestellt werden. Der Gewichtsanteil an A-Blöcken in den Blockcopolymeren beträgt vorzugsweise 40 bis 90 %.
Weitere geeignete Blockcopolymere sind beispielsweise Multiblockcopolymere, die erhältlich sind durch Reaktion eines Diols als Initiator (Block A) zunächst mit einem ersten Monomer (Block B), dann mit einem zweiten Monomer (Block C).
Geeignete Blockcopolymere sind beispielsweise auch sternförmige Blockcopolymere, die erhältlich sind, wenn anstelle des Diols ein Triol oder Tetrol als Initiator verwendet wird. Als trifunktionelle Initiatoren werden bevorzugt verwendet: kommerziell erhältliches Glycerin-ethoxylat bzw. Glycerin-propoxylat oder Glycerin-propoxylat-*b*-ethoxylat mit einem Molgewicht von 200 bis 6000 g/mol, besonders bevorzugt mit einem Molgewicht von 1000 und 3000 g/mol. Als tetrafunktionelle Initiatoren werden bevorzugt verwendet: kommerziell erhältliches Pentaerythrit-ethoxylat bzw. Pentaerythrit-propoxylat oder Pentaerythrit-propoxylat-*b*-ethoxylat mit einem Molgewicht von 200 bis 3000 g/mol, besonders bevorzugt mit einem Molgewicht von 500 und 2000 g/mol. Grundsätzlich sind auch Diblockcopolymere geeignet.
Die beschriebenen Blockcopolymere sind an den Enden bevorzugt mit Hydroxygruppen ausgestattet. Durch Derivatisierung mit einer chemisch vernetzbaren Gruppe lassen sich Endgruppen X erzielen, sodass man vernetzbare Makromere M erhält, z.B.:

P ---> Derivatisierung ---> M

bzw.

$A(B)_n$ ---> Derivatisierung ---> $A(B\text{-}X)_n$

Bevorzugte chemisch vernetzbare Gruppen sind ethylenisch ungesättigte, radikalisch polymerisierbare Gruppen. Radikalisch polymerisierbare Gruppen sind z.B. Vinyl-, Allyl-, Acrylat- oder Methacrylatgruppen. Sie können beispielsweise eingeführt werden durch Veresterung mit geeigneten ethylenisch ungesättigten Carbonsäuren oder durch Veretherung mit geeigneten ungesättigten Alkoholen. Durch Veresterung mit Methacrylsäurechlorid bzw. Acrylsäurechlorid oder Methacrylsäureestern bzw. Acrylsäureestern lassen sich Methacrylat- oder Acrylatendgruppen erzielen.
Zu Formgedächtnispolymeren vernetzbare Makromere bzw. Prepolymere im Sinne der Erfindung sind Polymere oder Oligomere, bei denen das Fixieren einer aufgeprägten permanenten Form dadurch erfolgt, dass einzelne Polymer- oder Oligomerstränge durch chemische Bindungen miteinander verknüpft werden. Die Vernetzung über chemische Bindungen kann über ionische oder kovalente Bindungen erfolgen. Die Vernetzungsreaktion kann eine beliebige chemische Reaktion, z.B. eine Salzbildungsreaktion, eine Kondensationreaktion, eine Additionsreaktion, eine Substitionsreaktion oder eine radikalisch oder photochemisch induzierte Reaktion sein. Die Vernetzungsreaktion kann mittels geeigneter Katalysatoren oder Initiatoren oder katalysatorfrei erfolgen. Sie kann durch eine geeignete Energiequelle ausgelöst werden, z.B. durch elektromagnetische Strahlung, Ultraschall, Wärme oder mechanische Energie. Eine Kombination zweier oder mehrerer Startverfahren kann gegebenenfalls zur Erhöhung der Effizienz oder der Geschwindigkeit der Vernetzungsreaktion eingesetzt werden.

Erfindungsgemäß geeignete kovalent vernetzbare Formgedächtnispolymere (Makromere M) weisen mindestens eine Übergangstemperatur $T_{trans}$ auf. Hierbei kann es sich um eine Schmelztemperatur $T_m$ oder um eine Glasübergangstemperatur $T_g$ handeln. Oberhalb von $T_{trans}$ weist das Polymer ein niedrigeres Elastizitätsmodul auf als unterhalb von $T_{trans}$. Das Verhältnis der Elastizitätsmodule unter- und oberhalb von $T_{trans}$ ist vorzugsweise mindestens 20. Die Übergangstemperatur $T_{trans}$ ist vorzugsweise größer als Raumtemperatur (20°C), insbesondere mindestens 30°C, besonders bevorzugt mindestens 40°C und ist diejenige Temperatur, bei deren Überschreiten die spontane Rückbildung der permanten Form aus der deformierten oder aus der temporären Form erfolgt.

Geeignete Makromere M sind z.B. solche der allgemeinen Formel

$$A(B\text{-}X)_n \qquad (II)$$

wobei A von einem n-valenten Poly- oder Oligoether, Kohlenwasserstoffen mit einem Molekulargewicht von mindestens 400 g/mol und n alkoholischen Hydroxygruppen, Oligoesterdiolen oder von einem Polyester einer Dicarbonsäure mit einem Diol, oder von einem Dimerdiol abgeleitet ist, B einen Poly(hydroxycarbonsäure)block, n eine Zahl größer gleich zwei und X eine reaktive, chemisch vernetzbare Gruppe bedeuten. A und B können dieselbe Bedutung wie in Formel (I) haben. X ist bevorzugt ausgewählt aus ethylenisch ungesättigten, radikalisch polymerisierbaren Gruppen, bevorzugt Acrylat- oder Methacrylatgruppen. n ist vorzugsweise 2, 3 oder 4. Bevorzugt sind X-B-A-B-X Triblockmakromere, insbesondere Blockpolymere mit einem einzigen Polyolblock A als Mittelblock, zwei endständigen Polyesterblöcken B aus Hydroxycarbonsäuren oder deren Lactonen sowie mit endständigen Acrylat- oder Methacrylatgruppen X.

Bevorzugte Blockcopolymere sind solche der allgemeinen Formel (IIa)

$$X1\text{-}[O\text{-}B1\text{-}C\,(=O)\,O\text{-}]_{n1}\,[Y\text{-}O\text{-}]_{n2}\,[C\,(=O)\,\text{-}B2\text{-}O\text{-}]_{n3}X2 \qquad (IIa)$$

X1 und X2 sind wie bei Formel (II) gleich oder verschieden und stehen für reaktive, chemisch vernetzbare Gruppen, vorzugsweise für ethylenisch ungesättigte, radikalisch polymerisierbare Gruppen. B1 und B2 sind gleich oder verschieden und stehen für verzweigte, cyclische oder lineare Alkylengruppen mit 1 bis 40, vorzusgweise mit 2 bis 20 C-Atomen. Y steht für eine verzweigte, cyclische oder lineare Alkylengruppe mit 2 bis 30 C-Atomen, vorzugsweise für Ethylen- und/ oder Propylengruppen, oder für einen Block aus Dimerdiol, Dimerdiololigoether oder Oligoesterdiol, und wobei n1, n2 und n3 gleiche oder verschiedene Zahlen größer oder gleich Null sind, wobei sowohl n2 als auch die Summe n1+n3 größer als Null sind.

Besonders bevorzugt sind Makromere der Formel (IIa), wobei X1 und X2 Acrylat oder Methacrylat sind, B1 und B2 für verzweigte oder lineare Alkylengruppen mit 2 bis 20 C-Atomen stehen, Y eine Ethylengruppe ist und n1, n2 und n3 größer Null sind und so gewählt sind, dass das Molekulargewicht des Makromers größer oder gleich 2000 ist. Geeignet sind z.B. Blockcopolymere mit einem Polyethylenglykolmittelblock und Endblöcken aus Polymilchsäure oder Poly-ε-caprolacton, wobei die Endblöcke an den Enden mit Methacrylatgruppen substituiert sind, der Mittelblock ein Molekulargewicht im Bereich von 1000 bis 20000. Vorzugsweise 2000 bis 10000 aufweist und das mittlere Molekulargewicht des Blockcopolymers von 2000 bis 50000, vorzugsweise von 3000 bis 25000 beträgt.

Erfindungsgemäße Zusammensetzungen für die Haarbehandlung enthalten mindestens eines der oben genannten Polymere M oder vernetzbaren Makromere M in einer Menge von vorzugsweise 0,01 bis 25 Gew.%, besonders bevorzugt von 0,1 bis 15 Gew.% in einem geeigneten flüssigen Medium. Die Zusammensetzung kann als Lösung, Dispersion, Emulsion, Suspension oder Latex vorliegen. Das flüssige Medium ist vorzugsweise kosmetisch akzeptabel und physiologisch unbedenklich.

In einer besonders bevorzugten Ausführungsform enthält die Zusammensetzung zusätzlich Makromere, die mit nur einer reaktiven Gruppe substituiert sind. Geeignete zusätzliche Makromere sind z.B. solche der allgemeinen Formel

$$R\text{-}(B')_n\text{-}X3 \qquad (III)$$

wobei R für einen monovalenten organischen Rest, X3 für eine reaktive, chemisch vernetzbare Gruppe und -(B')n- für ein divalentes, thermoplastisches Polymer- oder Oligomersegment steht. X3 ist vorzugsweise eine Acrylat- oder Methacrylatgruppe. Das Segment (B')n steht vorzugsweise für Polyalkylenglykole, deren Monoalkylether sowie deren Blockcopolymere, wobei die Alkylengruppen vorzugsweise Ethylen- oder Propylengruppen sind und die Alkylgruppen vorzugsweise 1 bis 30 C-Atome aufweisen.

Besonders bevorzugt sind endständig an einem Ende mit Acryl- oder Methacrylsäure veresterte Polyalkylenglykolmonoalkylether, wobei die Alkylengruppen vorzugseise Ethylen- oder Propylengruppen und die Alkylgruppen vorzugsweise C1- bis C30-Alkylgruppen sind, z.B. Poly(ethylenglykol)monoacrylat, Poly(propylenglykol)mono-acrylat und deren Monoalkylether.

Die erfindungsgemäße Zusammensetzung liegt im allgemeinen als Lösung oder Dispersion in einem geeigneten Lösungsmittel vor. Besonders bevorzugt sind wäßrige, alkoholische oder wäßrig-alkoholische Lösungsmittel. Geeignete Lösungsmittel sind z.B. aliphatische C1- bis C4-Alkohole oder ein Gemisch von Wasser mit einem dieser Alkohole. Es können jedoch auch andere organische Lösungsmittel eingesetzt werden, wobei insbesondere unverzweigte oder verzweigte Kohlenwasserstoffe wie Pentan, Hexan, Isopentan, cyklische Kohlenwasserstoffe wie Cyclopentan und Cyclohexan, organische lineare oder cyclische Ether, z.B. Tetrahydrofuran (THF) oder flüssige organische Ester, z.B. Ethylacetat zu nennen sind. Weiterhin sind auch Lösungsmittel auf Silikonbasis geeignet, insbesonders Silikonöle auf Basis linearer oder cyclischer Polydimethylsiloxane (Dimethicone oder Cyclomethicone). Die Lösungsmittel liegen bevorzugt in einer Menge von 0,5 bis 99 Gew.%, besonders bevorzugt in einer Menge von 40 bis 90 Gew.% vor.

Erfindungsgemäße Zusammensetzungen können zusätzlich 0,01 bis 25 Gew.% mindestens eines haarpflegenden, haarfestigenden und/oder haarfärbenden Wirkstoffes enthalten.

Haarfestigende Wirkstoffe sind insbesondere die bekannten, herkömmlichen filmbildenden und haarfestigenden Polymeren.

Das filmbildende und haarfestigende Polymer kann synthetischen oder natürlichen Ursprungs sein und nichtionischen, kationischen, anionischen oder amphoteren Charakter haben. Ein derartiger Polymerzusatz, der in Mengen von 0,01 bis 25 Gew.%, vorzugsweise 0,1 bis 20 Gew.%, besonders bevorzugt von 0,5 bis 15 Gew.% enthalten sein kann, kann auch aus einem Gemisch von mehreren Polymerern bestehen und durch den Zusatz von weiteren Polymeren mit verdickender Wirkung in seinen haarfestigenden Eigenschaften modifiziert werden. Unter filmbildenden, haarfestigenden Polymeren werden erfindungsgemäß solche Polymere verstanden, die bei Anwendung in 0,01 bis 5%-iger wäßriger, alkoholischer oder wäßrig-alkoholischer Lösung in der Lage sind, auf dem Haar einen Polymerfilm abzuscheiden und auf diese Weise das Haar zu festigen.

Als geeignete synthetische, nichtionische, filmbildende, haarfestigende Polymere können in dem erfindungsgemäßen Haarbehandlungsmittel Homopolymere des Vinylpyrrolidons, Homopolymere des N-Vinylformamids, Copolymerisate aus Vinylpyrrolidon und Vinylacetat, Terpolymere aus Vinylpyrrolidon, Vinylacetat und Vinylpropionat, Polyacrylamide, Polyvinylalkohole, oder Polyethylenglykole mit einem Molekulargewicht von 800 bis 20.000 g/mol eingesetzt werden. Unter den geeigneten synthetischen, filmbildenden anionischen Polymeren sind zu nennen Crotonsäure/Vinylacetat Copolymere und Terpolymere aus Acrylsäure, Ethylacrylat und N-t-Butylacrylamid. Natürliche filmbildende Polymere oder daraus durch chemische Umwandlung hergestellte Polymere können in dem erfindungsgemäßen Haarbehandlungsmittel ebenfalls eingesetzt werden. Bewährt haben sich niedermolekulares Chitosan mit einem Molekulargewicht von 30.000 bis 70.000 g/mol oder hochmolekulares Chitosan, organolösliche Derivate des Chitosans, Gemische aus Oligo-, Mono- und Disacchariden, chinesisches Balsamharz, Cellulosederivate wie Hydroxypropylcellulose mit einem Molekulargewicht von 30.000 bis 50.000 g/mol, oder Schellack in neutralisierter oder unneutralisierter Form. Auch amphotere Polymere können in dem erfindungsgemäßen Haarbehandlungsmittel eingesetzt werden. Geeignet sind z. B. Copolymere aus Octylacrylamid, t-Butylaminoethylmethacrylat sowie zwei oder mehr Monomeren aus der Gruppe Acrylsäure, Methacrylsäure und deren einfachen Estern. Unter den kationischen Polymeren, die erfindungsgemäß eingesetzt werden können, sind Copolymere des Vinylpyrrolidons mit quaternierten Derivaten des Dialkylaminoacrylats- und -methacrylats, wie beispielsweise mit Diethylsulfat quaternierte Vinylpyrrolidon/Dimethylaminomethacrylat Copolymere zu nennen. Weitere kationische Polymere sind beispielsweise das Copolymerisat des Vinylpyrrolidons mit Vinylimidazoliummethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcarprolactam, das quaternierte Ammoniumsalz, hergestellt aus Hydroxyethylcellulose und einem mit Trimethylammonium substituierten Epoxid, das Vinylpyrrolidon/ Methacrylamidopropyltrimethylammoniumchlorid Copolymer und diquaternäre Polydimethylsiloxane.

Die Konsistenz des erfindungsgemäßen Haarbehandlungsmittels kann durch den Zusatz von Verdickern erhöht werden. Hierfür sind beispielsweise Homopolymere der Acrylsäure mit einem Molekulargewicht von 2.000.000 bis 6.000.000 g/mol geeignet. Auch Copolymere aus Acrylsäure und Acrylamid (Natriumsalz) mit einem Molekulargewicht von 2.000.000 bis 6.000.000 g/mol, Sclerotium Gum und Copolymere der Acrylsäure und der Methacrylsäure sind geeignet.

Ein erfindungsgemäßes kosmetisches Mittel kann in verschiedenen Applikationsformen Anwendung finden, wie beispielsweise in Form einer Lotion, einer Sprühlotion, einer Creme, eines Gels, eines Gelschaums, eines Aerosolsprays, eines Nonaerosolsprays, eines Aerosolschaums, eines Nonaerosolschaums, einer O/W- oder W/O-Emulsion, einer Mikroemulsion oder eines Haarwachses.

Wenn das erfindungsgemäße Haarbehandlungsmittel in Form eines Aerosolsprays vorliegt, so enthält es zusätzlich 15 bis 85 Gew.%, bevorzugt 25 bis 75 Gew.% eines Treibmittels und wird in einem Druckbehälter mit Sprühkopf abgefüllt. Als Treibmittel sind niedere Alkane, wie z.B. n-Butan, Isobutan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie $N_2$, $N_2O$ und $CO_2$ sowie Gemische der vorstehend genannten Treibmittel geeignet.

Wenn das erfindungsgemäße Haarbehandlungsmittel in Form eines versprühbaren Non-Aerosol Haarsprays vorliegt, so wird es mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Wenn das erfindungsgemäße Haarbehandlungsmittel in Form eines Haarschaumes (Mousse) vorliegt, so enthält es mindestens eine übliche, hierfür bekannte schaumgebende Substanz. Das Mittel wird mit oder ohne Hilfe von Treibgasen oder chemischen Treibmitteln verschäumt und als Schaum in das Haar eingearbeitet und ohne Ausspülen im Haar belassen. Ein erfindungsgemäßes Produkt weist als zusätzliche Komponente eine Vorrichtung zum Verschäumen der Zusammensetzung auf. Unter Vorrichtungen zum Verschäumen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Wenn das erfindungsgemäße Haarbehandlungsmittel in Form eines Haargels vorliegt, so enthält es zusätzlich mindestens eine gelbildende Substanz in einer Menge von vorzugsweise 0,05 bis 10, besonders bevorzugt von 0,1 bis 2 Gew.%. Die Viskosität des Gels beträgt vorzugsweise von 100 bis 50.000 $mm^2$/s , besonders bevorzugt von 1.000 bis 15.000 $mm^2$/s bei 25°C, gemessen als dynamische Viskositätsmessung mit einem Bohlin Rheometer CS, Messkörper C25 bei einer Schergeschwindigkeit von 50 $s^{-1}$.

Wenn das erfindungsgemäße Haarbehandlungsmittel in Form eines Haarwachses vorliegt, so enthält es zusätzlich wasserunlösliche Fett- oder Wachsstoffe oder Stoffe, die der Zusammensetzung eine wachsähnliche Konsistenz verleihen, in einer Menge von vorzugsweise 0,5 bis 30 Gew.%. Geeignete wasserunlösliche Stoffe sind beispielsweise Emulgatoren mit einem HLB-Wert unterhalb von 7, Silikonöle, Silikonwachse, Wachse (z.B. Wachsalkohole, Wachssäuren, Wachsester, sowie insbesondere natürliche Wachse wie Bienenwachs, Carnaubawachs, etc.), Fettalkohole, Fettsäuren, Fettsäureester oder hochmolekulare Polyethylenglykole mit einem Molekulargewicht von 800 bis 20.000, vorzugsweise von 2.000 bis 10.000 g/mol.

Wenn das erfindungsgemäße Haarbehandlungsmittel in Form einer Haarlotion vorliegt, so liegt es als im wesentlichen nicht-viskose oder gering viskose, fließfähige Lösung, Dispersion oder Emulsion mit einem Gehalt an mindestens 10 Gew.%, vorzugsweise 20 bis 95 Gew.% eines kosmetisch verträglichen Alkohols vor. Als Alkohole können insbesondere die für kosmetische Zwecke üblicherweise verwendeten niederen C1- bis C4-Alkohole wie z.B. Ethanol und Isopropanol verwendet werden.

Wenn das erfindungsgemäße Haarbehandlungsmittel in Form einer Haarcreme vorliegt, so liegt es vorzugsweise als Emulsion vor und enthält entweder zusätzlich viskositätsgebende Inhaltsstoffe in einer Menge von 0,1 bis 10 Gew.% oder die erforderliche Viskosität und cremige Konsistenz wird durch Micellbildung mit Hilfe von geeigneten Emulgatoren, Fettsäuren, Fettalkoholen, Wachsen etc. in üblicher Weise aufgebaut.

In einer bevorzugten Ausführungsform ist das erfindungsgemäße Mittel in der Lage, gleichzeitig sowohl die Aufprägung einer abrufbaren Frisur als auch eine Haarfärbung zu ermöglichen. Das Mittel ist dann als färbendes Haarbehandlungsmittel wie z.B. als Farbfestiger, Färbecreme, Färbeschaum etc. formuliert. Es enthält dann mindestens einen färbenden Stoff. Hierbei kann es sich um organische Farbstoffe, insbesondere um sogenannte direktziehnde Farbstoffe oder auch um anorganische Pigmente handeln.

Die Gesamtmenge an Farbstoffen beträgt in dem erfindungsgemäßen Mittel etwa 0,01 bis 7 Gew.%, vorzugsweise etwa 0,2 bis 4 Gew.%. Für das erfindungsgemäße Mittel geeignete direktziehende Farbstoffe sind beispielsweise Triphenylmethanfarbstoffe, aromatische Nitrofarbstoffe, Azofarbstoffe, Chinonfarbstoffe, kationische oder anionische Farbstoffe. Weitere zur Haarfärbung bekannte und übliche Farbstoffe, die in dem erfindungsgemäßen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Geeignete haarfärbende Pigmente sind im Anwendungsmedium praktisch unlösliche Farbmittel und können anorganisch oder organisch sein. Auch anorganisch-organische Mischpigmente sind möglich. Bei den Pigmenten handelt es sich vorzugsweise nicht um Nanopigmente. Die bevorzugte Teilchengröße beträgt 1 bis 200 $\mu$m, insbesondere 3 bis

150 $\mu$m, besonders bevorzugt 10 bis 100 $\mu$m. Bevorzugt sind anorganische Pigmente.

Das erfindungsgemäße Haarbehandlungsmittel enthält vorzugsweise zusätzlich mindestens einen haarpflegenden Stoff in einer Menge von 0,01 bis 10, besonders bevorzugt von 0,05 bis 5 Gew.%. Bevorzugte haarpflegende Stoffe sind Silikonverbindungen sowie kationaktive Stoffe, die auf Grund von kationischen oder kationisierbaren Gruppen, insbesondere primären, sekundären, tertiären oder quaternären Amingruppen eine Substantivität zu menschlichem Haar aufweisen. Geeignete kationaktive Stoffe sind ausgewählt aus kationischen Tensiden, betainischen, amphoteren Tensiden, kationischen Polymeren, Silikonverbindungen mit kationischen oder kationisierbaren Gruppen, kationisch derivatisierten Proteinen oder Proteinhydrolysaten und Betain.

Geeignete Silikonverbindungen sind z.B. Polydimethylsiloxan (INCI: Dimethicon), $\alpha$-Hydro-$\omega$-hydroxypolyoxydimethylsilylen (INCI: Dimethiconol), cyclisches Dimethylpolysiloxan (INCI: Cyclomethicon), Trimethyl(octadecyloxy)silan (INCI: Stearoxy-trimethylsilan), Dimethylsiloxan/Glykol Copolymer (INCI: Dimethicon Copolyol), Dimethylsiloxan/Aminoalkylsiloxan Copolymer mit Hydroxyendgruppen (INCI: Amodimethicon), Monomethylpolysiloxan mit Laurylseitenketten und Polyoxyethylen- und/oder Polyoxypropylenendketten, (INCI: Laurylmethicon Copolyol), Dimethylsiloxan/Glykol Copolymeracetat (INCI: Dimethiconcopolyol Acetat), Dimethylsiloxan/Aminoalkylsiloxan Copolymer mit Trimethylsilylendgruppen (INCI: Trimethylsilylamodimethicon). Bevorzugte Silikonpolymere sind Dimethicone, Cyclomethicone und Dimethiconole. Auch Mischungen von Silikonpolymeren sind geeignet wie z.B. eine Mischung aus Dimethicon und Dimethiconol. Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Üblicherweise können dem erfindungsgemäßen Haarbehandlungsmittel weitere bekannte kosmetische Zusatzstoffe beigefügt werden, z.B. nichtfestigende, nichtionische Polymere wie Polyethylenglykole, nichtfestigende, anionische und natürliche Polymere sowie deren Mischungen in einer Menge von vorzugsweise 0,01 bis 50 Gew.%. Auch Parfümöle in einer Menge von 0,01 bis 5 Gew.%, Trübungsmittel wie Ethylenglykoldistearat in einer Menge von 0,01 bis 5 Gew.%, Netzmittel oder Emulgatoren aus den Klassen der anionischen, kationischen, amphoteren oder nichtionischen Tenside wie Fettalkoholsulfate, ethoxylierte Fettalkohole, Fettsäurealkanolamide wie die Ester der hydrierten Rizinusölfettsäuren in einer Menge von 0,1 bis 30 Gew.%, außerdem Feuchthaltemittel, Anfärbestoffe, Lichtschutzmittel, Antioxidantien und Konservierungsstoffe in einer Menge von 0,01 bis 10 Gew.%.

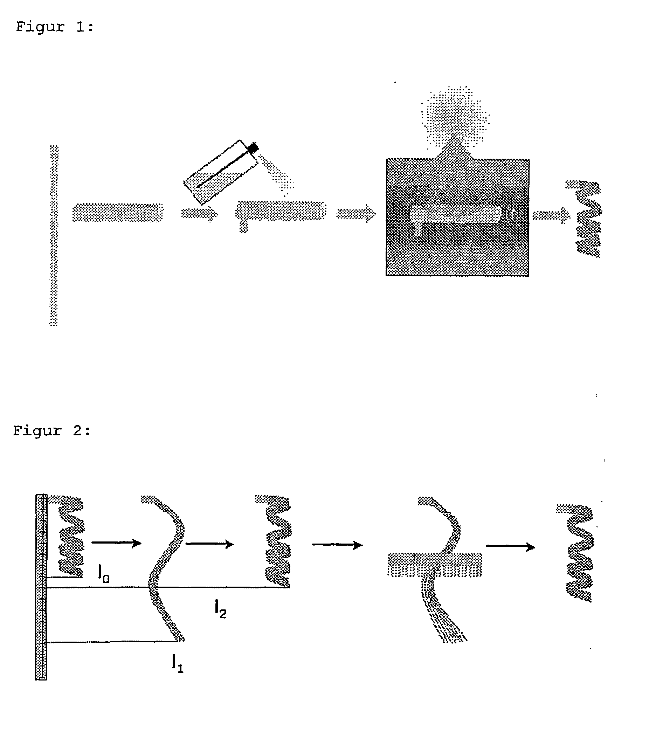

Figur 1 zeigt schematisch das Verfahren zur Herstellung einer abrufbaren permanenten Frisurenform. Eine Haarsträhne wird auf einen Wickelkörper gewickelt und mit einer erfindungsgemäßen, ein vernetzbares Macromer enthaltenden Lösung besprüht. Durch Bestrahlung mit einer geeigneten Energiequelle, z.B. einer UV-Lampe wird die gewünschte permanente Form fixiert. Zum Schluß wird der Wickelkörper entfernt.

Figur 2 zeigt die Deformation einer permanenten Frisurenform und Wiederherstellung der permanenten Form aus der temporären Form. Die Haarlocke in der permanenten Form hat die Länge $l_0$. Die Locke in der deformierten Form hat die Länge $l_1$. Die Locke in der wiederhergestellten Form hat die Länge $l_2$. Der Wiederherstellungsgrad (Recovery) berechnet sich nach: Recovery = $(l_1 - l_2) / (l_1 - l_0)$

Als Maß zur Beurteilung der Formgedächtniseigenschaften einer Zusammensetzung kann der Memory-Faktor dienen, in welchem sowohl die Umformbarkeit einer permanenten Frisurenform in eine temporäre Form (Formfaktor) als auch die Rückstellung der permanenten Form aus der temporären Form (Rückstellfaktor, Wiederherstellungsgrad) berücksichtigt werden. Wird von einer glatten Strähne ausgegangen, auf die eine Lockenform als permanente Form aufgeprägt wird und auf die anschließend eine zweite, glatte Form als temporäre Form aufgeprägt wird, so kann der Formfaktor bestimmt werden nach folgenden Kriterien:

| Grad der Glättung | Formfaktor |
|---|---|
| Durchgehend stark wellig | 0 |
| Schwach wellig von Ansatz bis Spitze | 1 |
| Haaransatz glatt, Spitze als Locke | 2 |
| Haaransatz glatt, Krümmung in der Spitze | 3 |
| Durchgehend glatt von Ansatz bis Spitze | 4 |

Der Rückstellfaktor kann bestimmt werden nach folgenden Kriterien:

| Grad der Rückstellung der permanenten Form | Rückstellfaktor |
|---|---|
| 0% | 0 |
| 30% | 1 |

(fortgesetzt)

| Grad der Rückstellung der permanenten Form | Rückstellfaktor |
|---|---|
| 40% | 2 |
| 50% | 3 |
| 60% | 4 |
| 75% | 5 |
| 100% | 6 |

Der Memory-Faktor M ergibt sich aus dem jeweiligen Formfaktor f, dem maximalen Formfaktor F=4, dem jeweiligen Rückstellfaktor r und dem maximalen Rückstellfaktor R=6 gemäß

$$M = (f/F) * (r/R) * 100$$

Der Memoryfaktor soll idealerweise nicht unter 20 liegen, bevorzugt mindestens 25 oder mindestens 30, besonders bevorzugt zwischen 35 und 100.
Die nachfolgenden Beispiele sollen den Gegenstand der Erfindung näher erläutern.

**Beispiele**

Die Herstellung der in den folgenden Beispielen verwendeten Triblockcopolymere erfolgt unter Verwendung der jeweiligen den Mittelblock bildenden Diolen als Initiator und Veresterung mit den jeweiligen die Endblöcke bildenden Polyestern mittels üblicher Veresterungsreaktionen. Die mit zwei Hydroxyendgruppen substituierten Triblockcopolymere werden mit Methacrylsäurechlorid zu den mit zwei Methacrylatgruppen substituierten Macromeren umgesetzt.

**Herstellung eines Oligoesterdiols**

Dicarbonsäure (1 mol) und Diol (1,2 bis 1,5 mol) werden bei 100 °C unter Rühren vermischt. Die Veresterungsreaktion wurde dann durch Zugabe von p-Toluolsulfonsäure (1 - 5 Gew.% bezogen auf die Gesamtmasse) gestartet. Nach Beendigung der Reaktion wird die Temperatur auf 40 °C abgekühlt und Methylenchlorid zum Lösen der Reaktionmasse zugegeben. Das Produkt wird in Methanol ausgefällt, mit Methanol gewaschen und im Vakuumtrockenschrank getrocknet. Die mittleren Molmassen werden mittels GPC (Kalibrierung mit Polystyrol) bestimmt.

**Beispiele 1 bis 112**

Es werden ca. 2-3%ige Zusammensetzungen der folgenden Blockcopolymere in Ethanol/Wasser (50:50) oder Ethanol/Wasser/Aceton (45:45:10) hergestellt.

| Mittelblock | Endblock | Mn | Beispiel Nr. | |
|---|---|---|---|---|
| | | | Endsubstituenten | |
| | | | Methacrylat | Hydroxygruppen |
| PEG-4k | PDL | 6000 | 1 | 57 |
| PEG-6k | PDL | 8000 | 2 | 58 |
| PEG-8k | PDL | 10000 | 3 | 59 |
| PEG-8k | PDL | 12000 | 4 | 60 |
| PEG-8k | P-LL-LA | 10000 | 5 | 61 |
| PEG-8k | P-LL-LA | 9000 | 6 | 62 |
| PEG-4k | P-DL-LA | 10000 | 7 | 63 |
| PEG-6k | P-DL-LA | 10000 | 8 | 64 |

(fortgesetzt)

| Mittelblock | Endblock | Mn | Beispiel Nr. | |
|---|---|---|---|---|
| | | | Endsubstituenten | |
| | | | Methacrylat | Hydroxygruppen |
| PEG-8k | P-DL-LA | 10000 | 9 | 65 |
| PEG-6k | PCL | 8000 | 10 | 66 |
| PEG-6k | PCL | 10000 | 11 | 67 |
| PEG-8k | PCL | 10000 | 12 | 68 |
| PEG-8k | PCL | 12000 | 13 | 69 |
| PEG-8k | PCL | 15000 | 14 | 70 |
| PEG-6k | P(CL-*co-DL*-LA) 30:10 | 10000 | 15 | 71 |
| PEG-6k | P(CL-*co-DL*-LA) 20:20 | 10000 | 16 | 72 |
| PEG-6k | P(CL-*co-DL*-LA) 10:30 | 10000 | 17 | 73 |
| PEG-6k | P(CL-*co-LL*-LA) 30:10 | 10000 | 18 | 74 |
| PEG-6k | P(CL-*co-LL*-LA) 20:20 | 10000 | 19 | 75 |
| PEG-6k | P(CL-*co-LL*-LA) 10:30 | 10000 | 20 | 76 |
| PEG-6k | P(CL-*co*-PDL) 30:10 | 10000 | 21 | 77 |
| PEG-6k | P(CL-*co*-PDL) 20:20 | 10000 | 22 | 78 |
| PEG-6k | P(CL-*co*-PDL) 10:30 | 10000 | 23 | 79 |
| PEG-6k | P(*LL*-LA-*co*-PDL) 30:10 | 10000 | 24 | 80 |
| PEG-6k | P(*LL*-LA-*co*-PDL) 20:20 | 10000 | 25 | 81 |
| PEG-6k | P(*LL*-LA-*co*-PDL) 10:30 | 10000 | 26 | 82 |
| PEG-6k | PCL-*b*-P-DL-LA | 10000 | 27 | 83 |
| PEG-6k | PCL-*b*-P-DL-LA | 12000 | 28 | 84 |
| PEG-6k | PCL-*b*-P-DL-LA | 15000 | 29 | 85 |
| PEG-8k | PCL-*b*-P-DL-LA | 10000 | 30 | 86 |
| PEG-8k | PCL-*b*-P-DL-LA | 12000 | 31 | 87 |
| PEG-8k | PCL-*b*-P-DL-LA | 15000 | 32 | 88 |
| PEG-6k | PCL-*b*-P-LL-LA | 10000 | 33 | 89 |
| PEG-6k | PCL-*b*-P-LL-LA | 12000 | 34 | 90 |
| PEG-6k | PCL-*b*-P-LL-LA | 15000 | 35 | 91 |

(fortgesetzt)

| Mittelblock | Endblock | Mn | Beispiel Nr. | |
|---|---|---|---|---|
| | | | Endsubstituenten | |
| | | | Methacrylat | Hydroxygruppen |
| PEG-8k | PCL-*b*-P-LL-LA | 10000 | 36 | 92 |
| PEG-8k | PCL-*b*-P-LL-LA | 12000 | 37 | 93 |
| PEG-8k | PCL-*b*-P-LL-LA | 15000 | 38 | 94 |
| PEG-6k | PCL-*b*-PPDL | 10000 | 39 | 95 |
| PEG-6k | PCL-*b*-PPDL | 12000 | 40 | 96 |
| PEG-6k | PCL-*b*-PDL | 15000 | 41 | 97 |
| PEG-8k | PCL-*b*-PPDL | 10000 | 42 | 98 |
| PEG-8k | PCL-*b*-PPDL | 12000 | 43 | 99 |
| PEG-8k | PCL-*b*-PDL | 15000 | 44 | 100 |
| PEG-6k | P-LL-LA-*b*-PPDL | 10000 | 45 | 101 |
| PEG-6k | P-LL-LA-*b*-PPDL | 12000 | 46 | 102 |
| PEG-6k | P-LL-LA-*b*-PPDL | 15000 | 47 | 103 |
| PEG-8k | P-LL-LA-*b*-PPDL | 10000 | 48 | 104 |
| PEG-8k | P-LL-LA-*b*-PPDL | 12000 | 49 | 105 |
| PEG-8k | P-LL-LA-*b*-PPDL | 15000 | 50 | 106 |
| PEG-6k | P-DL-LA-*b*-PPDL | 10000 | 51 | 107 |
| PEG-6k | P-DL-LA-*b*-PPDL | 12000 | 52 | 108 |
| PEG-6k | P-DL-LA-*b*-PPDL | 15000 | 53 | 109 |
| PEG-8k | P-DL-LA-*b*-PPDL | 10000 | 54 | 110 |
| PEG-8k | P-DL-LA-*b*-PPDL | 12000 | 55 | 111 |
| PEG-8k | P-DL-LA-*b*-PPDL | 15000 | 56 | 112 |

**Beispiele 113 bis 200**

Es werden ca. 2-3%ige Zusammensetzungen der folgenden Blockcopolymere in Ethanol/Wasser/Aceton (25: 25:50) hergestellt.

| Mittelblock | Endblock | Mn | Beispiel Nr. | |
|---|---|---|---|---|
| | | | Endsubstituenten | |
| | | | Methacrylat | Hydroxy |
| Dimerdiol | PPDL | 5000 | 113 | 157 |
| Dimerdiol | PPDL | 10000 | 114 | 158 |
| Dimerdiol | P-*LL*-LA | 5000 | 115 | 159 |
| Dimerdiol | P-*LL*-LA | 10000 | 116 | 160 |
| Dimerdiol | P-*DL*-LA | 5000 | 117 | 161 |
| Dimerdiol | P-*DL*-LA | 10000 | 118 | 162 |
| Dimerdiol | PCL | 5000 | 119 | 163 |

(fortgesetzt)

| Mittelblock | Endblock | Mn | Beispiel Nr. | |
| --- | --- | --- | --- | --- |
| | | | Endsubstituenten | |
| | | | Methacrylat | Hydroxy |
| Dimerdiol | PCL | 10000 | 120 | 164 |
| D-OEt 1000 | PPDL | 5000 | 121 | 165 |
| D-OEt 1000 | PPDL | 10000 | 122 | 166 |
| D-OEt 1000 | P-*LL*-LA | 5000 | 123 | 167 |
| D-OEt 1000 | P-*LL*-LA | 10000 | 124 | 168 |
| D-OEt 1000 | P-*DL*-LA | 5000 | 125 | 169 |
| D-OEt 1000 | P-*DL*-LA | 10000 | 126 | 170 |
| D-OEt 1000 | PCL | 5000 | 127 | 171 |
| D-OEt 1000 | PCL | 10000 | 128 | 172 |
| D-OEt 2000 | PPDL | 5000 | 129 | 173 |
| D-OEt 2000 | PPDL | 10000 | 130 | 174 |
| D-OEt 2000 | P-*LL*-LA | 5000 | 131 | 175 |
| D-OEt 2000 | P-*LL*-LA | 10000 | 132 | 176 |
| D-OEt 2000 | P-*DL*-LA | 5000 | 133 | 177 |
| D-OEt 2000 | P-*DL*-LA | 10000 | 134 | 178 |
| D-OEt 2000 | PCL | 5000 | 135 | 179 |
| D-OEt 2000 | PCL | 10000 | 136 | 180 |
| D-OEs 1000 | PPDL | 10000 | 137 | 181 |
| D-OEs 1000 | PCL | 10000 | 138 | 182 |
| D-OEs 1000 | P-LL-LA | 10000 | 139 | 183 |
| D-OEs 1000 | P-*DL*-LA | 10000 | 140 | 184 |
| D-OEs 2000 | PPDL | 10000 | 141 | 185 |
| D-OEs 2000 | PCL | 10000 | 142 | 186 |
| D-OEs 2000 | P-*LL*-LA | 10000 | 143 | 187 |
| D-OEs 2000 | P-*DL*-LA | 10000 | 144 | 188 |
| D-OEs 3000 | PPDL | 10000 | 145 | 189 |
| D-OEs 3000 | PCL | 10000 | 146 | 190 |
| D-OEs 3000 | P-*LL*-LA | 10000 | 147 | 191 |
| D-OEs 3000 | P-*DL*-LA | 10000 | 148 | 192 |
| D-OEs 4000 | PPDL | 10000 | 149 | 193 |
| D-OEs 4000 | PCL | 10000 | 150 | 194 |
| D-OEs 4000 | P-*LL*-LA | 10000 | 151 | 195 |
| D-OEs 4000 | P-*DL*-LA | 10000 | 152 | 196 |
| D-OEs 5000 | PPDL | 10000 | 153 | 197 |
| D-OEs 5000 | PCL | 10000 | 154 | 198 |
| D-OEs 5000 | P-*LL*-LA | 10000 | 155 | 199 |

(fortgesetzt)

| Mittelblock | Endblock | Mn | Beispiel Nr. | |
|---|---|---|---|---|
| | | | Endsubstituenten | |
| | | | Methacrylat | Hydroxy |
| D-OEs 5000 | P-*DL*-LA | 10000 | 156 | 200 |

**Beispiele: Haarbehandlung mit Triblockcopolymeren**

| | 1 | 2 | 3 | 4 | 5 |
|---|---|---|---|---|---|
| Blockcopolymer 1 | 3 Gew.% | | | | |
| Blockcopolymer 2 | | 3 Gew.% | | | |
| Blockcopolymer 3 | | | 3 Gew.% | | |
| Blockcopolymer 5 | | | | 3 Gew.% | |
| Blockcopolymer 6 | | | | | 3 Gew.% |
| Behandlung | UV | UV | UV | UV | UV |
| Lösungsmittel | Et/W 50/50 | Et/W 50/50 | Et/W 50/50 | Et/W 50/50 | Et/W 50/50 |
| $T_{trans}$ [°C] | 55, 83 | 55-60 | 55-60 | 40, 56 | 40, 56 |
| Memory-Faktor M[%] | 28 | 35 | 33 | 38 | 40 |

| | 6 | 7 | 8 | 9 | 10 |
|---|---|---|---|---|---|
| Blockpolymer 9 | 3 Gew.% | | | | |
| Blockpolymer 11 | | 3 Gew.% | | | |
| Blockpolymer 15 | | | 3 Gew.% | | |
| Blockpolymer 16 | | | | 3 Gew.% | |
| Blockpolymer 17 | | | | | 3 Gew.% |

| Behandlung | UV | UV | UV | UV | UV |
|---|---|---|---|---|---|
| Lösungsmittel | Et/W/Ac 45/45/10 | Et/W/Ac 45/45/10 | Et/W/Ac 45/45/10 | Et/W/Ac 45/45/10 | Et/W/Ac 45/45/10 |
| $T_{trans}$ [°C] | 50-55 | 50-55 | 50-55 | 50-55 | 50-55 |
| Memory-Faktor M | 30 | 40 | 28 | 28 | 25 |

| | 11 | 12 | 13 | 14 |
|---|---|---|---|---|
| Blockcopolymer 58 | 3 Gew.% | | | |
| Blockcopolymer 61 | | 3 Gew.% | | |
| Blockcopolymer 62 | | | 3 Gew.% | |
| Blockcopolymer 66 | | | | 3 Gew.% |
| Behandlung | Wärme | Wärme | Wärme | Wärme |
| Lösungsmittel | Et/W 50/50 | Et/W 50/50 | Et/W 50/50 | Et/W/Ac 45/45/10 |
| $T_{trans}$, $T'_{trans}$ [°C] | 57, 85 | 40, 57 | 40, 56 | 44, 53 |
| Memory-Faktor M | 25 | 42 | 40 | 30 |

| | 15 | 16 | 17 | 18 |
|---|---|---|---|---|
| Blockcopolymer 72 | 3 Gew.% | | | |
| Blockcopolymer 115 | | 3 Gew.% | | |
| Blockcopolymer 128 | | | 3 Gew.% | |
| Blockcopolymer 138 | | | | 3 Gew.% |
| Behandlung | Wärme | UV | UV | UV |
| Lösungsmittel | Et/W 50/50 | Et/W/AC 25/25/50 | Et/W/AC 25/25/50 | Et/W/AC 25/25/50 |
| $T_{trans}$ [°C] | 50-60 | 50 - 60 | 50-60 | 50-60 |
| Memory-Faktor M | 40 | 20 | 28 | 30 |

Ac = Aceton, W = Wasser, Et = Ethanol

**Haarbeschichtung und Herstellung der permanenten Form:**

Eine mit Wasser befeuchtete Haarsträhne einer Länge von 20 cm wird auf einen Haarwickler aufgedreht und die Polymerlösung wird aufgetragen (20-30 mg Polymer/g Haar). Die behandelte Strähne wird 30 min bei 70 °C mit oder ohne UV-Licht fixiert. Nach Abkühlung auf Raumtemperatur (etwa 25°C) wird der Haarwickler entfernt. Die gelockte Strähne (aufgeprägte permanente Form) hatte eine Länge von etwa 4,5 cm.

**Herstellung der temporären Form und Wiederherstellung der permanenten Form:**

Zur Herstellung der temporären Form (z.B. glatt) wird die gelockte Strähne auf ca. 55°C erwärmt, auf ihre ursprüngliche, volle Länge gestreckt (20 cm) und auf Raumtemperatur abgekühlt. Zur Wiederherstellung der permanenten Form wird die glatte Strähne auf ca. 55°C erwärmt. Die Strähne zog sich bei dieser Temperatur spontan zusammen zur permanenten Form (gelockt).

Zur erneuten Herstellung einer temporären Form (z.B. glatt) wird die gelockte Strähne noch einmal auf 55 °C erwärmt, auf ihre volle Länge (20 cm) gestreckt und auf Raumtemperatur abgekühlt.

**Beispiele haarkosmetischer Mittel**

**Beispiel 1:** Aerosol-Föhn-Lotion

| A | B | C | |
|---|---|---|---|
| 3,4 g | - | 1,7 g | Blockpolymer Nr. 5 |
| | 3,4 g | 1,7 g | Blockpolymer Nr. 61 |
| 0,20 g | 0,20 g | 0,20 g | Parfüm |
| 0,02 g | 0,02 g | 0,02 g | Baysilon® Öl PD 5 (Phenyl Trimethicone) |
| 10,00 g | 10,00 g | 10,00 g | Wasser |
| Ad 100 g | Ad 100 g | Ad 100 g | Ethanol |

Die Wirkstofflösung wird im Verhältnis 45 : 55 mit DME als Treibmittel in eine Aerosoldose abgefüllt.

**Beispiel 2:** Aerosol-Föhn-Lotion

| A | B | C | |
|---|---|---|---|
| 1,6 g | - | 3,2 g | Blockpolymer Nr. 1 |
| 1,6 g | 3,2 g | - | Blockpolymer Nr. 57 |
| 3,5 g | 3,5 g | 3,5 g | Amphomer® LV 71[1)] |
| 0,57 g | 0,57 g | 0,57 g | Aminomethylpropanol 95%ig |
| 0,20 g | 0,20 g | 0,20 g | Parfüm |
| 0,02 g | 0,02 g | 0,02 g | Baysilon® Öl PD 5 (Phenyl Trimethicone) |
| 10,00 g | 10,00 g | 10,00 g | Wasser |
| Ad 100 g | Ad 100 g | Ad 100 g | Ethanol |
| [1)] Octylacrylamid/Acrylsäure/Butylaminoethylmethacrylat/ Methylmethacrylat/Hydroxypropylmethacrylat Copolymer Die Wirkstofflösung wird im Verhältnis 45 : 55 mit DME als Treibmittel in eine Aerosoldose abgefüllt. | | | |

**Beispiel 3:** Aerosol-Föhn-Lotion

| A | B | C | |
|---|---|---|---|
| 3,4 g | 1,7 g | - | Blockpolymer Nr. 66 |
| - | 1,7 g | 3,4 g | Blockpolymer Nr. 10 |
| 3,335 g | 3,335 g | 3,335 g | Vinylacetat/Crotonsäure Copolymer (Luviset® CA 66) |
| 0,378 g | 0,378 g | 0,378 g | Aminomethylpropanol 95% |
| 0,20 g | 0,20 g | 0,20 g | Parfüm |
| 0,02 g | 0,02 g | 0,02 g | Baysilon® Öl PD 5 (Phenyl Trimethicone) |
| Ad 100 g | Ad 100 g | Ad 100 g | Ethanol |

Die Wirkstofflösung wird im Verhältnis 65:35 mit Propan/ Butan (2.7 bar) als Treibmittel in eine Aerosoldose abgefüllt.

**Beispiel 4:** Haarspray

| A | B | C | |
|---|---|---|---|
| 1,25 g | 2,5 g | - | Blockpolymer Nr. 65 |
| 1,25 g | - | 2,5 g | Blockpolymer Nr. 9 |
| 3,3 g | 3,3 g | 3,3 g | Luvimer® 100 P[1] |
| 0,844 g | 0,844 g | 0,844 g | Aminomethylpropanol 95% |
| 0,20 g | 0,20 g | 0,20 g | Parfüm |
| 0,02 g | 0,02 g | 0,02 g | Baysilon® Öl PD 5 (Phenyl Trimethicone) |
| 10,00 g | 10,00 g | 10,00 g | Wasser |
| Ad 100 g | Ad 100 g | Ad 100 g | Ethanol |
| 1) t-Butylacrylat/Ethylacrylat/ Methacrylsäure Copolymer Die Wirkstofflösung wurde im Verhältnis 45 : 55 mit DME als Treibmittel in eine Aerosoldose abgefüllt. | | | |

**Beispiel 5:** Aerosol-Schaumfestiger

| A | B | C | |
|---|---|---|---|
| 6 g | 3 g | - | Blockpolymer Nr. 63 |
| - | 3 g | 6 g | Blockpolymer Nr. 7 |
| 11,9 g | 11,9 g | 11,9 g | Ethanol |
| 0,14 g | 0,14 g | 0,14 g | Celquat® L200[1] |
| 0,2 g | 0,2 g | 0,2 g | Laureth-4 |
| 0,2 g | 0,2 g | 0,2 g | Panthenol |
| 0,2 g | 0,2 g | 0,2 g | Parfüm |
| 0,07 g | 0,07 g | 0,07 g | Cetyltrimethylammoniumbromid |
| 4 g | 4 g | 4 g | Propan |
| 4 g | 4 g | 4 g | Butan |
| Ad 100 g | Ad 100 g | Ad 100 g | Wasser |
| 1) Copolymer aus Hydroxyethylcellulose und Diallyldimethylammoniumchlorid; Polyquaternium-4 | | | |

**Beispiel 6:** Aerosol-Schaumfestiger

| A | B | C | |
|---|---|---|---|
| 3 g | 6 g | - | Blockpolymer Nr. 62 |

(fortgesetzt)

| A | B | C | |
|---|---|---|---|
| 3 g | - | 6 g | Blockpolymer Nr. 6 |
| 11,9 g | 11,9 g | 11,9 g | Ethanol |
| 0,3 g | 0,3 g | 0,3 g | Gafquat® 755 N[1) |
| 0,2 g | 0,2 g | 0,2 g | Laureth-4 |
| 0,2 g | 0,2 g | 0,2 g | Panthenol |
| 0,2 g | 0,2 g | 0,2 g | Parfüm |
| 0,07 g | 0,07 g | 0,07 g | Cetyltrimethylammoniumbromid |
| 4 g | 4 g | 4 g | Propan |
| 4 g | 4 g | 4 g | Butan |
| Ad 100 g | Ad 100 g | Ad 100 g | Wasser |
| 1) Vinylpyrrolidon/Dimethylaminoethylmethacrylat Copolymer | | | |

**Beispiel 7:** Aerosol-Schaumfestiger

| A | B | C | |
|---|---|---|---|
| 2 g | 4 g | - | Blockpolymer Nr. 64 |
| 2 g | - | 4,1 g | Blockpolymer Nr. 8 |
| 0,6 g | 0,6 g | 0,6 g | PVP/Vinylcaprolactam/DMAPA Acrylates Copolymer (Aquaflex® SF 40) |
| 0,07 g | 0,07 g | 0,07 g | Aminomethylpropanol 95% |
| 18,9 g | 18,9 g | 18,9 g | Ethanol |
| 0,4 g | 0,4 g | 0,4 g | PEG 25 PABA |
| 0,2 g | 0,2 g | 0,2 g | Laureth-4 |
| 0,2 g | 0,2 g | 0,2 g | Panthenol |
| 0,2 g | 0,2 g | 0,2 g | Parfüm |
| 0,07 g | 0,07 g | 0,07 g | Cetyltrimethylammoniumchlorid |
| 4 g | 4 g | 4 g | Propan |
| 4 g | 4 g | 4 g | Butan |
| Ad 100 g | Ad 100 g | Ad 100 g | Wasser |

**Beispiel 8:** Aerosol-Schaumfestiger

| A | B | C | |
|---|---|---|---|
| 2,2 | 5,5 | - | Blockpolymer Nr. 60 |
| 2,2 | - | 5,5 g | Blockpolymer Nr. 4 |
| 8,9 g | 8,9 g | 8,9 g | Ethanol |
| 0,45 g | 0,45 g | 0,45 g | Polyvinylpyrrolidon (PVP K 90) |

(fortgesetzt)

| A | B | C | |
|---|---|---|---|
| 0,4 g | 0,4 g | 0,4 g | PEG 25 PABA |
| 1,0 g | 1,0 g | 1,0 g | Abilquat® 3272 (Quaternium-80, 50%ig in Propylenglykol) |
| 0,15 g | 0,15 g | 0,15 g | Betain |
| 0,2 g | 0,2 g | 0,2 g | Parfüm |
| 0,07 g | 0,07 g | 0,07 g | Cetyltrimethylammoniumchlorid |
| 4 g | 4 g | 4 g | Propan |
| 4 g | 4 g | 4 g | Butan |
| Ad 100 g | Ad 100 g | Ad 100 g | Wasser |

**Beispiel 9:** Aerosol-Schaumfestiger

| A | B | C | |
|---|---|---|---|
| 1,8 | 3,5 | - | Blockpolymer Nr. 62 |
| 1,8 | - | 3,5 g | Blockpolymer Nr. 6 |
| 8,9 g | 8,9 g | 8,9 g | Ethanol |
| 0,45 g | 0,45 g | 0,45 g | Polyvinylpyrrolidon (PVP K 90) |
| 0,4 g | 0,4 g | 0,4 g | PEG 25 PABA |
| 1,0 g | 1,0 g | 1,0 g | Abilquat® 3272 (Quaternium-80, 50%ig in Propylenglykol) |
| 0,2 g | 0,2 g | 0,2 g | Parfüm |
| 0,07 g | 0,07 g | 0,07 g | Cetyltrimethylammoniumchlorid |
| 4 g | 4 g | 4 g | Propan |
| 4 g | 4 g | 4 g | Butan |
| Ad 100 g | Ad 100 g | Ad 100 g | Wasser |

**Beispiel 10:** Aerosol-Schaumfestiger

| A | B | C | |
|---|---|---|---|
| 2,8 g | 5,5 g | - | Blockpolymer Nr. 61 |
| 2,8 g | - | 5,5 g | Blockpolymer Nr. 5 |
| 8,9 g | 8,9 g | 8,9 g | Ethanol |
| 0,45 g | 0,45 g | 0,45 g | Polyvinylpyrrolidon (PVP K 30) |
| 0,4 g | 0,4 g | 0,4 g | PEG 25 PABA |
| 0,3 g | 0,3 g | 0,3 g | Celquat® L200 [1] |
| 0,2 g | 0,2 g | 0,2 g | Parfüm |
| 0,07 g | 0,07 g | 0,07 g | Cetyltrimethylammoniumbromid |
| 4 g | 4 g | 4 g | Propan |

(fortgesetzt)

| A | B | C | |
|---|---|---|---|
| 4 g | 4 g | 4 g | Butan |
| Ad 100 g | Ad 100 g | Ad 100 g | Wasser |
| [1] Copolymer aus Hydroxyethylcellulose und Diallyldimethylammoniumchlorid; Polyquaternium-4 | | | |

**Beispiel 11:** Non-Aerosol Föhnlotion

| A | B | C | |
|---|---|---|---|
| 2,3 g | 4,5 g | - | Blockpolymer Nr. 65 |
| 2,3 g | - | 4,5 g | Blockpolymer Nr. 9 |
| 0,45 g | 0,45 g | 0,45 g | Aristoflex® A[1] |
| 27 g | 27 g | 27 g | Ethanol |
| 0,7 g | 0,7 g | 0,7 g | PEG 25 PABA |
| 0,35 g | 0,35 g | 0,35 g | Panthenol |
| 0,1 g | 0,1 g | 0,1 g | Betain |
| 0,25 g | 0,25 g | 0,25 g | Parfüm |
| 0,21 g | 0,21 g | 0,21 g | PEG 40 Hydrogenated Castor Oil |
| 0,20 g | 0,20 g | 0,20 g | Cetyltrimethylammoniumchlorid |
| Ad 100 g | Ad 100 g | Ad 100 g | Wasser |
| [1] Vinylacetat/Crotonsäure/Polyethylenoxid Copolymer | | | |

Die Zusammensetzung kann in einer Verpackung mit Pumpsprühvorrichtung abgefüllt und als Non-Aerosol Spray angewendet werden.

**Beispiel 12:** Non-Aerosol Föhnlotion

| A | B | C | |
|---|---|---|---|
| 2,3 g | 4,5 g | - | Blockpolymer Nr. 62 |
| 2,3 g | - | 4,5 g | Blockpolymer Nr. 6 |
| 20 g | 20 g | 20 g | Ethanol |
| 0,7 g | 0,7 g | 0,7 g | PEG 25 PABA |
| 0,35 g | 0,35 g | 0,35 g | Panthenol |
| 0,1 g | 0,1 g | 0,1 g | Betain |
| 0,25 g | 0,25 g | 0,25 g | Parfüm |
| 0,21 g | 0,21 g | 0,21 g | PEG 40 Hydrogenated Castor Oil |
| 0,20 g | 0,20 g | 0,20 g | Cetyltrimethylammoniumbromid |
| Ad 100 g | Ad 100 g | Ad 100 g | Wasser |

Die Zusammensetzung kann in einer Verpackung mit Pumpsprühvorrichtung abgefüllt und als Non-Aerosol Spray angewendet werden.

**Beispiel 13:** Non-Aerosol Föhnlotion

| A | B | C | |
|---|---|---|---|
| 2,3 g | 4,5 g | - | Blockpolymer Nr. 65 |
| 2,3 g | - | 4,5 g | Blockpolymer Nr. 9 |
| 0,2 g | 0,2 g | 0,2 g | Polyquaternium-46 (Luviquat Hold) |
| 27 g | 27 g | 27 g | Ethanol |
| 0,7 g | 0,7 g | 0,7 g | PEG 25 PABA |
| 0,35 g | 0,35 g | 0,35 g | Panthenol |
| 0,1 g | 0,1 g | 0,1 g | Betain |
| 0,25 g | 0,25 g | 0,25 g | Parfüm |
| 0,21 g | 0,21 g | 0,21 g | PEG 40 Hydrogenated Castor Oil |
| 0,20 g | 0,20 g | 0,20 g | Cetyltrimethylammoniumchlorid |
| Ad 100 g | Ad 100 g | Ad 100 g | Wasser |

Die Zusammensetzung kann in einer Verpackung mit Pumpsprühvorrichtung abgefüllt und als Non-Aerosol Spray angewendet werden.

**Beispiel 14** Non-Aerosol Föhnlotion

| A | B | C | |
|---|---|---|---|
| 1,6 g | 3,1 g | - | Blockpolymer Nr. 69 |
| 1,6 g | - | 3,1 g | Blockpolymer Nr. 13 |
| 0,05 g | 0,05 g | 0,05 g | Celquat® L200 |
| 0,5 g | 0,5 g | 0,5 g | Polyvinylpyrrolidon/Vinylacetat Copolymer |
| 27 g | 27 g | 27 g | Ethanol |
| 0,7 g | 0,7 g | 0,7 g | PEG 25 PABA |
| 0,35 g | 0,35 g | 0,35 g | Panthenol |
| 0,1 g | 0,1 g | 0,1 g | Betain |
| 0,25 g | 0,25 g | 0,25 g | Parfüm |
| 0,21 g | 0,21 g | 0,21 g | PEG 40 Hydrogenated Castor Oil |
| 0,20 g | 0,20 g | 0,20 g | Cetyltrimethylammoniumbromid |
| Ad 100 g | Ad 100 g | Ad 100 g | Wasser |

Die Zusammensetzung kann in einer Verpackung mit Pumpsprühvorrichtung abgefüllt und als Non-Aerosol Spray angewendet werden.

**Beispiel 15:** Non-Aerosol Föhnlotion

| A | B | C | |
|---|---|---|---|
| 2 g | 4,1 g | - | Blockpolymer Nr. 62 |
| 2,g | - | 4,1 g | Blockpolymer Nr. 6 |
| 1,0 g | 1,0 g | 1,0 g | Blockpolymer Nr. 9 |
| 0,15 g | 0,15 g | 0,15 g | Polyquaternuim-46 (Luviquat Hold) |
| 27 g | 27 g | 27 g | Ethanol |
| 0,7 g | 0,7 g | 0,7 g | PEG 25 PABA |
| 0,35 g | 0,35 g | 0,35 g | Panthenol |
| 0,25 g | 0,25 g | 0,25 g | Parfüm |
| 0,21 g | 0,21 g | 0,21 g | PEG 40 Hydrogenated Castor Oil |
| 0,20 g | 0,20 g | 0,20 g | Cetyltrimethylammoniumchlorid |
| Ad 100 g | Ad 100 g | Ad 100 g | Wasser |

Die Zusammensetzung kann in einer Verpackung mit Pumpsprühvorrichtung abgefüllt und als Non-Aerosol Spray angewendet werden.

**Beispiel 16:** Nonaerosol Föhnlotion

| A | B | C | |
|---|---|---|---|
| 3 g | 6 g | - | Blockpolymer Nr. 62 |
| 3 g | - | 6,0 g | Blockpolymer Nr. 6 |
| 2,0 g | 2,0 g | 2,0 g | Vinylcaprolactam/VP/Dimethylaminoeth yl Methacrylate Copolymer (Advantage® S) |
| 28,5 g | 28,5 g | 28,5 g | Ethanol |
| 0,25 g | 0,25 g | 0,25 g | Parfüm |
| 0,20 g | 0,20 g | 0,20 g | Cetyltrimethylammoniumchlorid |
| Ad 60 g | Ad 60 g | Ad 60 g | Wasser |

Die Zusammensetzung kann in einer Verpackung mit Pumpsprühvorrichtung abgefüllt und als Non-Aerosol Spray angewendet werden.

**Beispiel 17:** Sprühfestiger

| A | B | C | |
|---|---|---|---|
| 3 g | 6 g | - | Blockpolymer Nr. 62 |
| 3 g | - | 6,0 g | Blockpolymer Nr. 6 |
| 2,0 g | 2,0 g | 2,0 g | Amphomer® |
| 28,5 g | 28,5 g | 28,5 g | Ethanol |

(fortgesetzt)

| A | B | C | |
|---|---|---|---|
| 0,6 g | 0,6 g | 0,6 g | Aminomethylpropanol 95% |
| 0,25 g | 0,25 g | 0,25 g | Parfüm |
| 0,20 g | 0,20 g | 0,20 g | Cetyltrimethylammoniumbromid |
| Ad 60 g | Ad 60 g | Ad 60 g | Wasser |

Die Zusammensetzung kann in einer Verpackung mit Pumpsprühvorrichtung abgefüllt und als Non-Aerosol Spray angewendet werden.

**Beispiel 18:** Sprühfestiger

| A | B | C | |
|---|---|---|---|
| 1,5 g. | 3 g | - | Blockpolymer Nr. 62 |
| 1,5 g | - | 3,0 g | Blockpolymer Nr. 6 |
| 0,65 g | 0,65 g | 0,65 g | Amphomer® |
| 0,2 g | 0,2 g | 0,2 g | Celquat® L200 |
| 28,5 g | 28,5 g | 28,5 g | Ethanol |
| 0,6 g | 0,6 g | 0,6 g | Aminomethylpropanol 95 % |
| 0,25 g | 0,25 g | 0,25 g | Parfüm |
| 0,20 g | 0,20 g | 0,20 g | Cetyltrimethylammoniumchlorid |
| Ad 60 g | Ad 60 g | Ad 60 g | Wasser |

Die Zusammensetzung kann in einer Verpackung mit Pumpsprühvorrichtung abgefüllt und als Non-Aerosol Spray angewendet werden.

**Beispiel 19:** Sprühgel

| A | B | C | |
|---|---|---|---|
| 2,6 g | 5,2 g | - | Blockpolymer Nr. 63 |
| 2,6 g | - | 5,2 g | Blockpolymer Nr. 7 |
| 20 g | 20 g | 20 g | Ethanol |
| 0,1 g | 0,1 g | 0,1 g | Aminomethylpropanol 95% |
| 0,2 g | 0,2 g | 0,2 g | PEG-40 |
| 0,2 g | 0,2 g | 0,2 g | Parfüm |
| 1,5 g | 1,5 g | 1,5 g | Carbomer |
| Ad 70 g | Ad 70 g | Ad 70 g | Wasser |

Die Zusammensetzung kann in einer Verpackung mit Pumpsprühvorrichtung abgefüllt und als Non-Aerosol Spray angewendet werden.

**Beispiel 20:** Sprühgel

| A | B | C | |
|---|---|---|---|
| 2 g | 4 g | - | Blockpolymer Nr. 52 |
| 2 g | - | 4,0 g | Blockpolymer Nr. 6 |
| 3,0 g | 3,0 g | 3,0 g | VP/VA COPOLYMER (Luviskol VA 64) |
| 18 g | 18 g | 18 g | Ethanol |
| 0,1 g | 0,1 g | 0,1 g | Aminomethylpropanol 95% |
| 0,2 g | 0,2 g | 0,2 g | PEG-40 |
| 0,2 g | 0,2 g | 0,2 g | Parfüm |
| 1,5 g | 1,5 g | 1,5 g | Carbomer |
| Ad 70 g | Ad 70 g | Ad 70 g | Wasser |

Die Zusammensetzung kann in einer Verpackung mit Pumpsprühvorrichtung abgefüllt und als Non-Aerosol Spray angewendet werden.

**Beispiel 21:** Pump-Schaumfestiger

| A | B | C | |
|---|---|---|---|
| 2,8 g | 5,5 g | - | Blockpolymer Nr. 52 |
| 2,8 g | - | 5,5 g | Blockpolymer Nr. 6 |
| 8,9 g | 8,9 g | 8,9 g | Ethanol |
| 0,2 g | 0,2 g | 0,2 g | Cocamidopropyl Hydroxysultaine |
| 0,2 g | 0,2 g | 0,2 g | Cetyltrimethylammoniumchlorid |
| 0,15 g | 0,15 g | 0,15 g | Parfüm |
| 0,1 g | 0,1 g | 0,1 g | Zitronensäure |
| 0,1 g | 0,1 g | 0,1 g | Betain |
| Ad 100 g | Ad 100 g | Ad 100 g | Wasser |

**Beispiel 22:** Pump-Schaumfestiger

| A | B | C | |
|---|---|---|---|
| 2 g | 4 g | - | Blockpolymer Nr. 65 |
| 2 g | - | 4,0 g | Blockpolymer Nr. 9 |
| 0,3 g | 0,3 g | 0,3 g | Celquat® L200 |
| 8,9 g | 8,9 g | 8,9 g | Ethanol |
| 0,2 g | 0,2 g | 0,2 g | Cocamidopropyl Hydroxysultaine |
| 0,2 g | 0,2 g | 0,2 g | Cetyltrimethylammoniumchlorid |

(fortgesetzt)

| A | B | C | |
|---|---|---|---|
| 0,15 g | 0,15 g | 0,15 g | Parfüm |
| 0,1 g | 0,1 g | 0,1 g | Zitronensäure |
| 0,1 g | 0,1 g | 0,1 g | Betain |
| Ad 100 g | Ad 100 g | Ad 100 g | Wasser |

**Beispiel 23:** Pump-Schaumfestiger

| A | B | C | |
|---|---|---|---|
| 2,2 g | 4,4 g | - | Blockpolymer Nr. 64 |
| 2,2 g | - | 4,4 g | Blockpolymer Nr. 8 |
| 2,4 g | 2,4 g | 2,4 g | Advantage® LC-E [1] |
| 8,9 g | 8,9 g | 8,9 g | Ethanol |
| 0,4 g | 0,4 g | 0,4 g | Cocamidopropyl Hydroxysultaine |
| 0,15 g | 0,15 g | 0,15 g | Parfüm |
| 0,1 g | 0,1 g | 0,1 g | Zitronensäure |
| Ad 100 g | Ad 100 g | Ad 100 g | Wasser |
| [1] Vinyl Caprolactam/VP/Dimethylaminoethyl Methacrylate Copolymer | | | |

**Beispiel 24:** Pump-Schaumfestiger

| A | B | C | |
|---|---|---|---|
| 2,6 g | 5,1 g | - | Blockpolymer Nr. 70 |
| 2,6 g | - | 5,1 g | Blockpolymer Nr. 14 |
| 0,35 g | 0,35 g | 0,35 g | Luvimer® 30 E[1] |
| 0,05 g | 0,05 g | 0,05 g | Chitosan (C XII) |
| 0,89 g | 0,89 g | 0,89 g | Aquaflex® SF 40 [2] |
| 0,06 g | 0,06 g | 0,06 g | Aminomethylpropanol 95% |
| 8,9 g | 8,9 g | 8,9 g | Ethanol |
| 0,4 g | 0,4 g | 0,4 g | Cocamidopropyl Hydroxysultaine |
| 0,15 g | 0,15 g | 0,15 g | Parfüm |
| 0,1 g | 0,1 g | 0,1g | Zitronensäure |
| Ad 100 g | Ad 100 g | Ad 100 g | Wasser |
| [1] t-Butlyacrylat/Ethylacrylat/Methacrylsäure Copolymer [2] PVP/Vinyl Caprolactam/DMAPA Acrylates Copolymer | | | |

In den vorgenannten haarkosmetischen Mitteln kann jeweils alternativ oder zusätzlich ein anderes der Blockpolymere Nr. 1 bis 200 enthalten sein.
Die in den Beispielen verwendeten Abkürzungen bedeuten:

PEG-4k, PEG-6k, PEG-8k: Polyethylenglykol mit Molekulargewicht von 4000, 6000 bzw. 8000
PDL: Pentadecalacton; Lacton der 15-Hydroxypentadecansäure
PPDL: Poly(pentadecalacton), Poly(15-hydroxypentadecansäure)
P-*LL*-LA: Poly(L-Milchsäure)
P-*DL*-LA: Poly(DL-Milchsäure)
PCL: Poly($\varepsilon$-caprolacton)
P(CL-*co*-LA): Poly(e-caprolacton-*co-DL*-Milchsäure)
PCL-*b*-PPDL: Poly(e-caprolacton)-*block*-Poly(pentadecalacton)
PEG(4k)-DMA, PEG(8k)-DMA, PEG(10k)-DMA:
Poly(ethylenglykol)-dimethacrylat
PLGA(7k)-DMA: Poly(L-lactid-co-glycolid)-dimethacrylat
PCl(10k)-DMA: Poly($\varepsilon$-caprolacton)-dimethacrylat
D-OEt 1000: Dimerdiol-oligoether Mn = 1000 (Sovermol 909)
D-OEt 2000: Dimerdiol-oligoether Mn = 2000 (Sovermol 910)
D-OEs 1000: Oligoester-diol $M_n$ = 1000
aus Glycerolmonostearat und Azelainsäure
D-OEs 2000: Oligoester-diol $M_n$ = 2000
aus Hydroxystearylalkohol und Adipinsäure
D-OEs 3000: Oligoester-diol $M_n$ = 3000
aus Hydroxystearylalkohol und Azelainsäure
D-OEs 4000: Oligoester-diol $M_n$ = 4000
aus Hydroxystearylalkohol und Azelainsäure D-OEs 5000: Oligoester-diol $M_n$ = 5000
aus Hydroxystearylalkohol und Azelainsäure

## Patentansprüche

1. Verfahren zur Haarbehandlung, wobei

   - eine Zusammensetzung, welche mindestens ein Formgedächtnispolymer P und/oder mindestens ein vernetzbare Makromer M, welches nach Vernetzung ein Formgedächtnispolymer bildet, enthält, auf das Haar aufgebracht wird,
   wobei das Polymer P und das Makromer M gebildet werden aus Blockpolymeren aus jeweils mindestens einem ersten Block, bei dem es sich um ein Polyol handelt, ausgewählt aus Polyethern, Oligoethern, Kohlenwasserstoffen mit einem Molekulargewicht von mindestens 400 g/mol und mindestens zwei alkoholischen Hydroxygruppen, Oligoesterdiolen und Polyestern aus Dicarbonsäuren mit Diolen
   und mindestens zwei weiteren Blöcken, bei denen es sich um Polyester von Hydroxycarbonsäuren oder deren Lactonen handelt,
   wobei das Makromer M

       a) vernetzbare Bereiche enthält, die durch chemische Bindungen vernetzbar sind und
       b) thermoplastische Bereiche enthält, die nicht chemisch vernetzbar sind und
       c) das nach Vernetzung gebildete Formgedächtnispolymer mindestens eine Übergangstemperatur $T_{trans}$ aufweist;

   und wobei das Polymer P

       a) mindestens ein durch physikalische Wechselwirkung vernetzbares hartes Segment mit einer ersten Übergangstemperatur $T_{trans}$, die oberhalb Raumtemperatur liegt, und
       b) mindestens ein weiches Segment mit einer zweiten Übergangstemperatur $T_{trans}$, welche unterhalb von $T_{trans}$ liegt, aufweist;

   - vorher, gleichzeitig oder anschließend das Haar in eine bestimmte Form gebracht wird und
   - anschließend die Form durch chemische Vernetzung des Makromers unter Ausbildung eines Formgedächtnispolymers und/oder unter physikalischer Vernetzung des Polymers P fixiert wird; wobei die programmierte Frisur (permanente Form) auf eine Temperatur oberhalb $T_{trans}$ erwärmt wird,
   - das Haar in eine zweite (temporäre) Form gebracht wird und
   - die zweite Form durch Abkühlen auf eine Temperatur unterhalb $T_{trans}$ fixiert wird.

**2.** Verfahren nach Anspruch 1, **dadurch gekennzeichnet, dass** das Macromer M die Formel A(B-X)$_n$ und das Polymer P die Formel A(B)$_n$ aufweisen, wobei A von einem n-valenten Poly- oder Oligoether, Kohlenwasserstoffen mit einem Molekulargewicht von mindestens 400 g/mol und n alkoholischen Hydroxygruppen, Oligoesterdiolen oder von einem Polyester einer Dicarbonsäure mit einem Diol, abgeleitet ist, B einen Poly(hydroxycarbonsäure)block, n eine Zahl größer gleich zwei und X eine reaktive, chemisch vernetzbare Gruppe bedeuten.

**3.** Verfahren nach Anspruch 2, **dadurch gekennzeichnet, dass**

A ausgewählt ist aus Polyalkylenglykolethern von mehrwertigen Alkoholen, Poly(tetrahydrofuran), Dimerdiol, Dimerdiololigoethern, Oligoesterdiolen,
B ausgewählt ist aus Poly($\epsilon$-caprolacton), Poly(pentadecalacton), Polylactiden, Polyglycoliden, Poly(lactid-co-glycolid),
X ausgewählt ist aus ethylenisch ungesättigten, radikalisch polymerisierbaren Gruppen und
n 2, 3 oder 4 ist.

**4.** Verfahren nach Anspruch 1, **dadurch gekennzeichnet, dass** das Makromer M die allgemeine Formel

$$X1-O-[B1-C(=O)O-]_{n1}[Y-O-]_{n2}[C(=O)-B2-O-]_{n3}X2$$

oder das Formgedächtnispolymer P die allgemeine Formel

$$HO-[B1-C(=O)O-]_{n1}[Y-O-]_{n2}[C(=O)-B2-O-]_{n3}H$$

aufweisen, wobei X1 und X2 gleich oder verschieden sind und für reaktive, chemisch vernetzbare Gruppen stehen, B1 und B2 gleich oder verschieden sind und für verzweigte, cyclische oder lineare Alkylengruppen mit 1 bis 40 C-Atomen stehen, Y für eine verzweigte, cyclische oder lineare Alkylengruppe mit 2 bis 30 C-Atomen oder für einen Polyesterblock aus Dicarbonsäure und Diol steht und n1, n2 und n3 gleiche oder verschiedene Zahlen größer Null sind.

**5.** Verfahren nach Anspruch 4, **dadurch gekennzeichnet, dass** X1 und X2 ethylenisch ungesättigte, radikalisch polymerisierbare Gruppen sind, B1 und B2 für verzweigte, cyclische oder lineare Alkylengruppen mit 2 bis 20 C-Atomen stehen und Y für Ethylen- und/oder Propylengruppen steht.

**6.** Verfahren nach Anspruch 5, **dadurch gekennzeichnet, dass** X1 und X2 Acrylat oder Methacrylat sind, B1 und B2 für verzweigte oder lineare Alkylengruppen mit 2 bis 20 C-Atomen stehen, Y eine Ethylengruppe ist und n1, n2 und n3 so gewählt sind, dass das Molekulargewicht des Makromers bzw. Polymers größer oder gleich 2000 ist.

**7.** Verfahren zur Wiederherstellung einer zuvor programmierten Frisur (permanente Form), wobei eine Frisur in einer temporären Form gemäß Anspruch 1 auf eine Temperatur oberhalb T$_{trans}$ erwärmt wird.

**8.** Verfahren nach einem der vorhergehenden Ansprüche, **dadurch gekennzeichnet, dass** in der Zusammensetzung zusätzlich ein Makromer mit nur einer end- oder seitenständigen, chemisch reaktiven Gruppe enthalten ist.

**9.** Verfahren nach Anspruch 8, **dadurch gekennzeichnet, dass** das zusätzliche Makromer ausgewählt ist aus Verbindungen der allgemeinen Formel

$$R-(X')_n-A3$$

wobei R für einen monovalenten organischen Rest, A3 für eine reaktive, chemisch vernetzbare Gruppe und -(X')n- für ein divalentes, thermoplastisches Polymer- oder Oligomersegment steht.

**10.** Verfahren nach Anspruch 9, **dadurch gekennzeichnet, dass** das zusätzliche Makromer ausgewählt ist aus mit einer Acrylat- oder Methacrylatgruppe substituierten Polyalkylenglykolen, deren Monoalkylether sowie deren Blockcopolymere.

**11.** Verfahren nach Anspruch 1, **dadurch gekennzeichnet, dass** mindestens ein Formgedächtnispolymer P eingesetzt wird und die Formgebung der Haare unter Erwärmung auf eine Temperatur von mindestens T$_{trans}$ erfolgt und dass die anschließende Fixierung der Haarform durch Abkühlung auf eine Temperatur unterhalb T'$_{trans}$ erfolgt.

**12.** Verfahren nach Anspruch 11, **dadurch gekennzeichnet, dass** das Formgedächtnispolymer P einen Kristallinitätsgrad von 3 bis 80% aufweist und dass das Verhältnis der Elastizitätsmodule unterhalb und oberhalb von $T_{trans}$ mindestens 20 beträgt.

**Claims**

**1.** Method for hair treatment, wherein

- a composition that contains at least one shape memory polymer P and / or at least one cross-linkable macromer M that forms a shape memory polymer after cross-linking is applied to the hair,
wherein the polymer P and the macromer M are each formed from block polymers having at least a first block which is a polyol selected from polyethers, oligoethers, hydrocarbons having a molecular weight of at least 400 g/mol and at least two alcoholic hydroxy groups, oligoester diols and polyesters of dicarboxylic acids with diols and at least two additional blocks, which are polyesters of hydroxycarboxylic acids or their lactones,
wherein the macromer M

a) contains cross-linkable regions that are cross-linkable through chemical bonds and
b) thermoplastic regions that are not chemically cross-linkable and
c) the shape memory polymer formed after cross-linking has at least one transition temperature $T_{trans}$;

and wherein the polymer P

a) has at least one hard segment that can be cross-linked by means of physical interaction, said hard segment having a first transition temperature $T'_{trans}$, which is above room temperature, and
b) has at least one soft segment with a second transition temperature $T_{trans}$ which is lower than $T'_{trans}$;

- before, simultaneously or subsequently the hair is arranged into a defined shape and
- the shape is subsequently fixed by chemically cross-linking the macromer, while forming a shape memory polymer and / or using physical cross-linking of the polymer P;

wherein the programmed hairstyle (permanent shape) is heated to a temperature above $T_{trans}$,

- the hair is brought into a second (temporary) shape and
- the second shape is fixed by means of cooling to a temperature below $T_{trans}$.

**2.** Method according to Claim 1, **characterised in that** the macromer M has the formula $A(B-X)_n$ and the polymer P has the formula $A(B)_n$, wherein A is derived from an n-valent polyether or oligoether, hydrocarbons having a molecular weight of at least 400 g/mol and n alcoholic hydroxyl groups, oligoester diols or from a polyester of a dicarboxylic acid with one diol, B represents a poly(hydroxycarboxylic acid) block and n represents a number greater than or equal to two and X represents a reactive, chemically cross-linkable group.

**3.** Method according to Claim 2, **characterised in that**

A is selected from polyalkylene glycol ethers of polyvalent alcohols, poly(tetrahydrofurane), dimerdiol, dimerdiol oligoethers, oligoester diols,
B is selected from poly($\varepsilon$-caprolactone), poly(pentadecalactone), polylactides, polyglycolides, poly(lactide-co glycolide),
X is selected from ethylenically unsaturated, radically polymerizable groups and
n is 2, 3 or 4.

**4.** Method according to Claim 1, **characterised in that** the macromer M has the general formula

$$X1\text{-}0\text{-}[B1\text{-}C\ (=O)O\text{-}]_{n1}[Y\text{-}O]_{n2}[C(=O)\text{-}B2\text{-}O\text{-}]_{n3}X2$$

or the shape memory polymer P has the general formula

$$HO\text{-}[B1\text{-}C(=O)O\text{-}]_{n1}\ [Y\text{-}O]_{n2}[C(=O)\text{-}B2\text{-}O\text{-}]_{n3}H$$

wherein X1 and X2 are the same or different and represent reactive, chemically cross-linkable groups, B1 and B2 are the same or different and stand for branched, cyclic or linear alkylene groups with 1 to 40 C atoms, Y stands for a branched, cyclic or linear alkylene group with 2 to 30 C atoms or for a polyester block of dicarboxylic acid and diol and n1, n2 and n3 are the same or different numbers greater than zero.

5. Method according to Claim 4, **characterised in that** X1 and X2 are ethylenically unsaturated, radically polymerizable groups, B1 and B2 stand for branched, cyclic or linear alkylene groups with 2 to 20 C atoms and Y stands for ethylene groups and / or propylene groups.

6. Method according to Claim 5, **characterised in that** X1 and X2 are acrylate or methacrylate, B1 and B2 stand for branched or linear alkylene groups with 2 to 20 C atoms, Y stands for an ethylene group and where n1, n2 and n3 are selected in such a way that the molecular weight of the macromer or polymer is greater than or equal to 2,000.

7. Method for the recovery of a previously programmed hairstyle (permanent shape), wherein a hairstyle in a temporary shape according to Claim 1 is heated to a temperature above $T_{trans}$.

8. Method according to one of the preceding claims, **characterised in that** the composition additionally contains a macromer with only one chemically reactive group located at a terminal position or a side position.

9. Method according to Claim 8, **characterised in that** the additional macromer is selected from compounds with the general formula

$$R-(X')_n A3$$

wherein R designates a monovalent organic residue, A3 designates a reactive, chemically cross-linkable group and -(X')n- designates a divalent, thermoplastic polymer or oligomer segment

10. Method according to Claim 9, **characterised in that** the additional macromer is selected from polyalkylene glycols substituted with an acrylate group or methacrylate group or from its monoalkyl ethers and block copolymers.

11. Method according to Claim 1, **characterised in that** at least one shape memory polymer P is used and that the shaping of the hair takes place under heating to a temperature of at least $T_{trans}$ and that the subsequent fixing of the hair shape takes place by cooling to a temperature below $T'_{trans}$.

12. Method according to Claim 11, **characterised in that** the shape memory polymer P has a degree of crystallinity of from 3 to 80% and that the ratio of the moduli of elasticity below and above $T_{trans}$ is at least 20.

**Revendications**

1. Procédé de traitement des cheveux, dans lequel

- une composition qui contient au moins un polymère à mémoire de forme P et/ou au moins un macromère réticulable M qui forme, après réticulation, un polymère à mémoire de forme, est appliquée sur les cheveux, le polymère P et le macromère M étant constitués de polymères blocs comprenant respectivement au moins un premier bloc qui est un polyol choisi parmi des polyéthers, des oligo-éthers, des hydrocarbures présentant un poids moléculaire d'au moins 400 g/mole et au moins deux groupes hydroxy alcooliques, des oligo-ester-diols et des polyesters d'acides dicarboxyliques avec des diols, et au moins deux autres blocs qui sont des polyesters d'acides hydroxycarboxyliques ou leurs lactones, le macromère M contenant

a) des zones réticulables qui sont réticulables par des liaisons chimiques, et
b) des zones thermoplastiques qui ne sont pas réticulables chimiquement, et
c) le polymère à mémoire de forme formé après réticulation présentant au moins une température de transition $T_{trans}$ ;

et le polymère P présentant

a) au moins un segment dur réticulable par interaction physique avec une première température de transition $T'_{trans}$ qui est supérieure à la température ambiante, et

b) au moins un segment souple avec une deuxième température de transition $T_{trans}$ qui est inférieure à $T'_{trans}$ ;

- une forme déterminée est donnée aux cheveux avant, pendant ou après, et
- la forme est ensuite fixée par réticulation chimique du macromère en produisant un polymère à mémoire de forme et/ou par réticulation physique du polymère P ;
- la coiffure prévue (forme permanente) est portée à une température supérieure à $T_{trans}$,
- les cheveux sont amenés sous deuxième forme (temporaire) et
- la deuxième forme est fixée par refroidissement à une température inférieure à $T_{trans}$.

**2.** Procédé selon la revendication 1, **caractérisé en ce que** le macromère M présente la formule $A(B-X)_n$ et le polymère P présente la formule $A(B)_n$, A étant issu d'un poly- ou oligo-éther de valence n, d'un hydrocarbure présentant un poids moléculaire d'au moins 400 g/mole et n groupes hydroxy alcooliques, d'un oligoester-diol ou d'un polyester d'un acide dicarboxylique avec un diol, B représentant un bloc poly(acide hydroxycarboxylique), n étant un nombre supérieur ou égal à 2 et X étant un groupe réactif, chimiquement réticulable.

**3.** Procédé selon la revendication 2, **caractérisé en ce que**

A est choisi parmi les éthers de polylalkylène glycols de polyalcools, le poly(tétrahydrofurane), le dimère diol, les oligoéthers de dimère diol, les oligoesters diols,

B est choisi parmi le poly($\varepsilon$-caprolactone), le poly(pentadécalactone), le polylactidène, le polyglycolidène, le poly(lactide-co-glycolide),

X est choisi parmi les groupes éthyléniquement insaturés polymérisables par vie radicalaire, et

n étant 2, 3 ou 4.

**4.** Procédé selon la revendication 1, **caractérisé en ce que** le macromère M présente la formule générale

$$X1-O-[B1-C(=O)O-]_{n1}[Y-O-]_{n2}[C(=O)-B2-O-]_{n3}X2$$

ou le polymère à mémoire de forme P présente la formule générale

$$HO-[B1-C(=O)O-]_{n1}[Y-O-]_{n2}[C(=O)-B2-O-]_{n3}H$$

dans lesquelles X1 et X2 sont identiques ou différents et désignent des groupes réactifs, réticulables chimiquement, B1 et B2 sont identiques ou différents et désignent des groupes alkylène ramifiés, cycliques ou linéaires comportant 1 à 40 atomes de carbone, Y est un groupe alkylène ramifié, cyclique ou linéaire comportant 2 à 30 atomes de carbone ou un bloc polyester d'un acide dicarboxylique et d'un diol et n1, n2 et n3 sont des nombres identiques ou différents supérieurs à zéro.

**5.** Procédé selon la revendication 4, **caractérisé en ce que** X1 et X2 sont des groupes éthyléniquement insaturés, polymérisables par voie radicalaire, B1 et B2 sont des groupes alkylène ramifiés, cycliques ou linéaires comportant 2 à 20 atomes de carbone et Y désigne des groupes éthylène et/ou propylène.

**6.** Procédé selon la revendication 5, **caractérisé en ce que** X1 et X2 sont un acrylate ou un méthacrylate, B1 et B2 désignent des groupes alkylène ramifiés ou linéaires comportant 2 à 20 atomes de carbone, Y est un groupe éthylène et n1, n2 et n3 sont choisis de telle sorte que le poids moléculaire du macromère ou du polymère soit supérieur ou égal à 2000.

**7.** Procédé de rétablissement d'une coiffure programmée au préalable (forme permanente), dans lequel une coiffure ayant une forme temporaire est chauffée selon la revendication 1 à une température supérieure à $T_{trans}$.

**8.** Procédé selon l'une des revendications précédentes, **caractérisé en ce que** dans la composition, est présent un macromère additionnel comportant un seul groupe chimiquement réactif situé au niveau terminal ou latéral.

**9.** Procédé selon la revendication 8, **caractérisé en ce que** le macromère additionnel est choisi parmi les composés de formule générale

R-(X')$_n$-A3

où R désigne un reste organique monovalent, A3 un groupe réactif, chimiquement réticulable et -(X')n un segment de polymère ou d'oligomère, divalent, thermoplastique.

10. Procédé selon la revendication 9, **caractérisé en ce que** le macromère additionnel est choisi parmi des polyalkylène-glycols substitués par un groupe acrylate ou méthacrylate, leurs monoalkyléthers et leurs copolymères blocs.

11. Procédé selon la revendication 1, **caractérisé en ce qu'**au moins un polymère à mémoire de forme P est utilisé et la mise en forme des cheveux est effectuée par chauffage à une température d'au moins T'$_{trans}$ et **en ce que** la fixation consécutive de la forme des cheveux est effectuée par refroidissement à une température inférieure à T'$_{trans}$.

12. Procédé selon la revendication 11, **caractérisé en ce que** le polymère à mémoire de forme P présente un degré de cristallinité de 3 à 80 % et **en ce que** le rapport des modules d'élasticité au-dessus et en-dessous de Ttrans est d'au moins 20.

Figur 1:

Figur 2:

**IN DER BESCHREIBUNG AUFGEFÜHRTE DOKUMENTE**

**In der Beschreibung aufgeführte Patentdokumente**

- JP 4041416 A
- DE 1768313 B

**In der Beschreibung aufgeführte Nicht-Patentliteratur**

- **E. SAGARIN.** Cosmetics, Science and Technology. Interscience Publishers Inc, 1957, 503 ff
- **H. JANISTYN.** Handbuch der Kosmetika und Riechstoffe. 1973, vol. 3, 388 ff
- **K. SCHRADER.** Grundlagen und Rezepturen der Kosmetika. 1989, 782-815